(19) Europäisches Patentamt
European Patent Office
Office européen des brevets

(11) Publication number: **0 356 223 A2**

(12) # EUROPEAN PATENT APPLICATION

(21) Application number: **89308555.5**

(22) Date of filing: **23.08.89**

(51) Int. Cl.[5]: **C 07 K 5/02**
A 61 K 37/64, C 07 D 237/22,
C 07 D 311/06,
C 07 D 207/333

(30) Priority: **24.08.88 US 236495 28.03.89 US 328645**

(43) Date of publication of application:
**28.02.90 Bulletin 90/09**

(84) Designated Contracting States:
**AT BE CH DE ES FR GB GR IT LI LU NL SE**

(71) Applicant: **MERCK & CO. INC.**
**126, East Lincoln Avenue P.O. Box 2000**
**Rahway New Jersey 07065-0900 (US)**

(72) Inventor: **Desolms, S. Jane**
**735 Port Indian Road**
**Norristown PA 19403 (US)**

**Huff, Joel R.**
**738 Bergey Mill Road**
**Lederach PA 19450 (US)**

**Vacca, Joseph P.**
**766 Eisenhauer Drive**
**Telford PA 18969 (US)**

**Sigal, Irving S.**
**Deceased (US)**

**Darke, Paul L.**
**434 West Walnut Street**
**North Wales PA 19454 (US)**

**Young, Steven D.**
**128 Mark Drive**
**Lansdale PA 19446 (US)**

(74) Representative: **Hesketh, Alan, Dr. et al**
**European Patent Department Merck & Co., Inc. Terlings Park Eastwick Road**
**Harlow Essex, CM20 2QR (GB)**

(54) **HIV protease inhibitors useful for the treatment of aids.**

(57) Compounds of the form,
A-G-B-B-J
wherein A is an amine protecting group not useful or commonly employed in peptide synthesis, G a dipeptide isostere, B an amino acid or analog thereof, and J a small terminal group are described. These compounds are useful in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 356 223 A2

**Description**

## HIV PROTEASE INHIBITORS USEFUL FOR THE TREATMENT OF AIDS

The present invention is concerned with compounds which inhibit the protease encoded by human immunodeficiency virus (HIV) or pharmaceutically acceptable salts thereof and are of value in the prevention of infection by HIV, treatment of infection by HIV and the treatment of the resulting acquired immune deficiency syndrome (AIDS). It also relates to pharmaceutical compositions containing the compounds and to a method of use of the present compounds and other agents for the treatment of AIDS.

### BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing or precursor polyproteins by a virally encoded protease to generate mature viral proteins required for virus assembly and function. Interruption of this processing appears to prevent the production of normally infectious virus. For example, Crawford, S. et al., J. Virol., 53, 899, 1985, demonstrated that genetic deletion mutations of the protease in murine leukemia virus which prevent processing of precursor structural proteins results in non-infectious viral particles. Unprocessed structural proteins also have been observed in clones of non-infectious HIV strains isolated from human patients. These results suggest that inhibition of the HIV protease represents a viable method for the treatment of AIDS or the prevention of infection by HIV.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267(1985); Power, M.D. et al., Science, 231, 1567(1986); Pearl, L.H. et al. Nature 329, 351(1987)]. Applicants demonstrate that the compounds of this invention are inhibitors of HIV protease.

### BRIEF DESCRIPTION OF THE INVENTION

Compounds of formula I, as herein defined, are disclosed. These compounds are useful in the inhibition of HIV protease, the prevention of infection by HIV, the treatment of infection by HIV and in the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also disclosed

### ABBREVIATIONS

| Designation | Amino Acid/Residue |
|---|---|
| Ala | D- or L-alanine |
| Arg | D- or L-arginine |
| Cal (Cha) | ß-cyclohexylalanine |
| Cys | D- or L-cysteine |
| Gly | glycine |
| His | D- or L-histidine |
| Ile | L-isoleucine |
| Leu | D- or L-leucine |
| Lys | D- or L-lysine |
| Met | D- or L-methionine |

| Designation | Amino Acid/Residue |
|---|---|
| Nle | L-norleucine |
| Nva | L-norvaline |
| Orn | D- or L-ornithine |
| Ph | phenyl |
| Phe | D- or L-phenylalanine |
| Pro | D- or L-proline |
| Sar | sarcosine (N-methylglycine) |
| Ser | D- or L-serine |
| Sta | statine, (3S,4S)-4-amino-3-hydroxy-6-methylheptanoic acid |
| Thr | D- or L-threonine |
| Trp | D- or L-tryptophan |
| Tyr | D- or L-tyrosine |
| Val | L-valine |
| | Protecting Group |
| BOC (Boc) | *t*-butyloxycarbonyl |
| BOM | benzyloxymethyl |
| CBZ (Cbz) | benzyloxycarbonyl(carbo-benzoxy) |
| DNP | 2,4-dinitrophenyl |
| IPOC | isopropoxycarbonyl |
| OMe | methyl ether (methoxy), except when it immediately follows an amino acid residue abbreviation and it represents methyl ester. |

| Designation | Reagent |
|---|---|
| OEt | ethoxy, except when it immediately follows an amino acid residue abbreviation and it represents ethyl ester |
| TBDMS-Cl | *t*-butyldimethylsilyl chloride |
| | **Activating Group** |
| HBT(HOBT) | 1-hydroxybenzotriazole hydrate |
| OMs | methane sulfonyloxy |
| | **Condensing Agent** |
| DCCI (DCC) | dicyclohexylcarbodiimide |
| DPPA | diphenylphosphorylazide |
| $(BOC)_2O$ | di-*t*-butyl dicarbonate |
| DEAD | diethyl azodicarboxylate |
| MCPBA | 3-chloropenoxybenzoic acid |
| TEA | triethylamine |
| TFA | trifluoroacetic acid |
| | **Coupling Reagents** |
| BOP reagent | benzotriazol-1-yloxytris-(dimethyl-amino)phosphonium hexafluorophosphate |
| BOP-Cl | bis(2-oxo-3-oxazolidinyl) phosphinic chloride |
| DSO | N,N'-disuccinimidyl oxalate |

| Designation | Reagent |
|---|---|
| EDC | 1-ethyl-3-(3-dimethylaminopropyl)carbodiimide hydrochloride |

DETAILED DESCRIPTION OF THE INVENTION AND PREFERRED EMBODIMENTS

This invention is concerned with the use of Compounds of Formula I, combinations thereof, or pharmaceutically acceptable salts thereof, in the inhibition of HIV protease, the prevention of infection by HIV, the treatment of infection by HIV and in the treatment of the resulting acquired immune deficiency syndrome (AIDS). Compounds of Formula I are defined as follows:

A-G-B-B-J    I,

wherein A is

1)

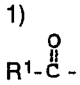 wherein $R^1$ is

a) $C_{1-6}$ alkyl either unsubstituted or substituted with one or more of

i) $C_{1-4}$ alkyl;

ii) hydroxy;

iii) carboxy;

iv) halo wherein halo is F, Cl, Br, or I; except no halo on carbon adjacent to carbonyl;

v) amino;

vi) $C_{1-3}$ alkoxycarbonyl;

vii) $C_{1-3}$ alkoxy;

viii) $-CONR^2R^3$ wherein $R^2$ and $R^3$ are the same or different and are hydrogen, $C_{1-5}$ alkyl or $C_{1-5}$ alkoxyalkyl or joined together either directly to form a 5-7 membered heterocycle such as pyrrolidinyl or piperidyl, or through a heteroatom selected from N, O, and S, to form a 6-membered heterocycle with the nitrogen to which they are attached such as morpholinyl, piperazinyl, or N-$C_{1-3}$ alkylpiperazinyl;

ix) $-NR^2R^3$;

x) 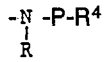 wherein,

R is hydrogen or $C_{1-4}$ alkyl,

P is

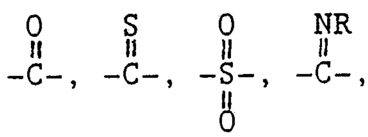

and

$R^4$ is H, $C_{1-3}$ alkyl, $C_{1-4}$ alkoxy, or $NR^2R^3$;

xi) $C_{3-7}$ cycloalkyl or $C_{6-10}$ aryl;

xii) 5 or 6 membered heterocycle, unsubstituted or substituted with -OH, NH, or $C_{1-4}$ alkyl; or

xiii) aryl of 6-10 carbon atoms, either unsubstituted or substituted with one or more of

(a) halo,

(b) hydroxy,

(c) $C_{1-3}$ alkoxy,

(d) $C_{1-3}$ alkyl,

(e) $-NR_2$, wherein R is defined above,

(f)

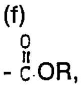,

(g)

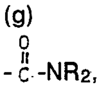,

(h) $-SO_2NR_2$,

(i) $-CH_2NR_2$,

(j)

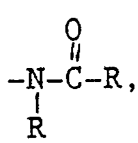

or

(k) 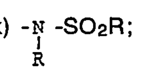;

xiv) $-OSiR^3(R^2)_2$;

b) aryl of 6-10 carbon atoms, either unsubstituted or substituted with one or more of

i) $C_{1-4}$ alkyl,

ii) $C_{1-3}$ alkoxy,
iii) hydroxy, or
iv) halo;
v) $-NR_2$;
vi)
$-\overset{O}{\overset{\|}{C}}OR$,
vii)
$-\overset{O}{\overset{\|}{C}}NR_2$,
viii) $-SO_2NR_2$,
ix) $-CH_2NR_2$,
x) -NRCOR, or
xi) $-NRSO_2R$;
c) 5 or 6 membered heterocycle as defined below;
2) $R^1-SO_2-$, except $R^1$ is not aryl,
3)

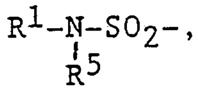

wherein
$R^5$ is H or $C_{1-5}$ alkyl or joined together with $R^1$ either directly to form 5-7 membered heterocycle such as pyrrolidinyl or piperidinyl, or through a heteroatom selected from N, O, and S, to form a 6-membered heterocycle with the nitrogen to which they are attached such as morpholinyl, piperazinyl, or N-$C_{1-3}$ alkyl-piperazinyl;
4)

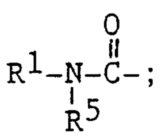

5)
$R^1-S-\overset{O}{\overset{\|}{C}}-$;
6) $(R^{1}-)_q$ wherein q is 1 or 2.
G is

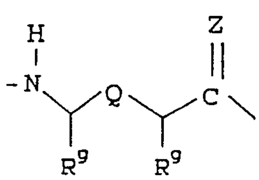

or

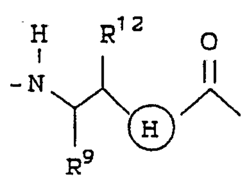

,

wherein Z is O, S, or HH, and
$R^9$ is independently
1) hydrogen;
2)

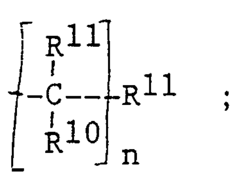

;

3) -OR, wherein R is H, or $C_{1-4}$ alkyl
4) $-NR_2$,
5) $C_{1-4}$ alkylene-$R^{11}$;
wherein n is 0-5 and $R^{10}$ is independently
a) hydrogen,

b) hydroxy, or
c) $C_{1-4}$-alkyl;
$R^{11}$ is
a) hydrogen
b) aryl, unsubstituted or substituted with one or more of
i) halo,
ii) hydroxy,
iii) $-NH_2$, $-NO_2$, -NHR, or $-NR_2$,
wherein R is
H, or $C_{1-4}$ alkyl,
iv) $C_{1-4}$ alkyl,
v) $C_{1-3}$ alkoxy,
vi) -COOR,
vii)
$-\overset{O}{\overset{\|}{C}}NR_2$,
viii) $-CH_2NR_2$,
ix)
$-CH_2NH\overset{O}{\overset{\|}{C}}R$,
x) CN,
xi) $CF_3$,
xii)
$-NH\overset{O}{\overset{\|}{C}}R$,
xiii) aryl $C_{1-3}$ alkoxy,
xiv) aryl,
xv) $-NRSO_2R$,
xvi) $-OP(O)(OR_x)_2$ wherein $R_x$ is H or aryl, or
xvii)
$-O-\overset{O}{\overset{\|}{C}}-C_{1-4}$ alkyl substituted with one or more of amine or quaternary amine;
c) 5 or 6 membered heterocycle including up to 3 heteroatoms selected from N, O, and S, such as imidazolyl, thiazolyl, furyl, oxazolyl, piperidyl, thiadiazolyl, piperazinyl, pyridyl, or pyrazinyl, any of which heterocycle may be unsubstituted or substituted with one or more of
i) halo,
ii) hydroxy,
iii) $-NH_2$, -NHR, $-NR_2$,
iv) $C_{1-4}$ alkyl,
v) $C_{1-3}$ alkoxy,
vi) -COOR,
vii)
$-\overset{O}{\overset{\|}{C}}NR_2$,
viii) $-CH_2NR_2$,
ix)
$-NH\overset{O}{\overset{\|}{C}}R$,
x) -CN,
xi) $CF_3$,
xii) $-NHSO_2R$,
xiii) $-OP(O)(OR_x)_2$ wherein $R_x$ is H or aryl, or
xiv)
$-O-\overset{O}{\overset{\|}{C}}-C_{1-4}$ alkyl substituted with one or more of amine or quaternary amine;
d) $C_{1-6}$ alkyl or $C_{1-6}$ alkenyl, unsubstituted or substituted with one or more of
i) hydroxy,
ii) $C_{1-4}$ alkyl,
iii) $-NH_2$, -NHR, $-NR_2$,
iv)
$-NH\overset{NH}{\overset{\|}{CH}}$ ,
v)
$-NH-\overset{NH}{\overset{\|}{C}}-NH_2$,

vi) -COOH,
vii)
$-\overset{O}{\overset{\|}{C}}OR$,
viii) -SR, or arylthio
ix) $-SO_2NHR$,
x) $C_{1-4}$ alkyl sulfonyl amino or aryl sulfonyl amino,
xi) -CONHR,
xii)
$-NH\overset{O}{\overset{\|}{C}}R$,
xiii) -OR,
xiv) aryl $C_{1-3}$ alkoxy or,
xv) aryl;
e) $C_{3-7}$ cycloalkyl unsubstituted or substituted with one or more of
i) hydroxy,
ii) $C_{1-4}$alkyl,
iii) $-NH_2$, -NHR, $-NHR_2$,
iv)
$-NH-\overset{NH}{\overset{\|}{CH}}$ ,
v)
$-NH-\overset{NH}{\overset{\|}{C}}-NH_2$,
vi) -COOH,
vii)
$-\overset{O}{\overset{\|}{C}}-OR$,
viii) -SR,
ix) $-SO_2NH_2$,
x) alkyl sulfonylamino or aryl sulfonylamino,
xi) -CONHR, or
xii)
$-NH\overset{O}{\overset{\|}{C}}R$;
f) a 5- to 7-membered carbocyclic or 7- to 10-membered bicyclic carbocyclic ring which is either saturated or unsaturated, such as cyclopentane, cyclohexane, indan, norbornane, or naphthanene, the carbocyclic ring being unsubstituted or substituted with one or more of
i) halo
ii) -OR, wherein R is H or $C_{1-4}$ alkyl,
iii)
$-\overset{O}{\overset{\|}{C}}OR$,
iv)
$-\overset{O}{\overset{\|}{C}}NR_2$,
v) $-CH_2NR_2$,
vi) $-SO_2NR_2$ or $-S(O)_y$ R wherein y is 0,1 or 2,
vii) $-NR_2$,
viii)
$-NH\overset{O}{\overset{\|}{C}}R$,
ix) $C_{1-4}$ alkyl,
x) phenyl,
xi) $-CF_3$, or
xii)
$-\overset{R}{\overset{|}{N}}-SO_2R$;
g) benzofuryl; indolyl; azabicyclo $C_{7-11}$ cycloalkyl; or benzopiperidinyl;
$R^{12}$ is -OH or $-NHR^{13}$, wherein $R^{13}$ is -H,
$-\overset{O}{\overset{\|}{C}}H$, $-C_{1-4}$-alkyl or -COOR; and
Ⓗ is
1) $C_{3-7}$ cycloalkyl either unsubstituted or substituted with one or more of
a) $C_{1-4}$alkyl,

b) hydroxy,
c) $-NR_2$,
d) -COOR,
e) CONHR,
f) $-NHSO_2R$,
g) $-NH\overset{O}{\overset{\|}{C}}R$,
h) aryl,
i) aryl substituted with $C_{1-4}$ alkyl,
j) heterocycle, or
k) heterocycle substituted with $C_{1-4}$ alkyl;
2) phenyl either unsubstituted or substituted with one or more of
a) hydroxy,
b) -OR,
c) $-NHR^{13}$,
d) -COOR,
e) $-\overset{O}{\overset{\|}{C}}NR_2$, or
f) $-NH\overset{O}{\overset{\|}{C}}R$;
3) 5 to 7-membered heterocycle such as imidazolyl, thiazolyl, furyl, oxazolyl, piperidyl, piperazinyl, pyridyl, or pyrazinyl, any of which heterocycle may be unsubstituted or substituted with one or more of
i) halo,
ii) hydroxy,
iii) $NR_2$, or
iv) $C_{1-4}$ alkyl;
Q is

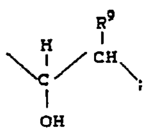 ; 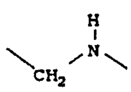 ; 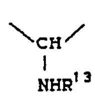 ; 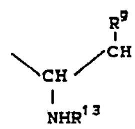 ; 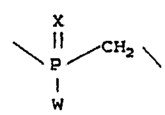 , 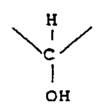 .

wherein $R^9$ and $R^{13}$ are defined above; X is O, S, or NH; and
W is
1) OH,
2) $NH_2$,
3) OR, or
4) NHR;
B is, independently, absent, or

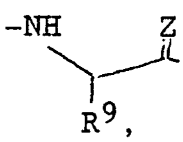

J is
1) $YR^{14}$ wherein:
Y is
O or NH, and
$R^{14}$ is
a) H;
b) $C_{1-6}$ alkyl, unsubstituted or substituted with one or more of
i) $-NR^2_2$,
ii) -OR,
iii) $-NHSO_2C_{1-4}$ alkyl,
iv) $-NHSO_2$ aryl, or $-NHSO_2$ (dialkylaminoaryl),
v) $-CH_2OR$,
vi) $C_{1-4}$ alkyl,
vii) $-\overset{O}{\overset{\|}{C}}OR$,

viii)
$-\overset{O}{\overset{\|}{C}}NR_2$,
ix)

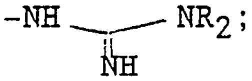
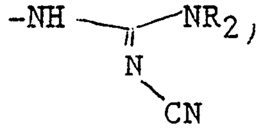

x)
$-NH\overset{O}{\overset{\|}{C}}R$,
xi)

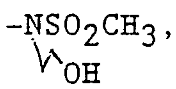

xii)

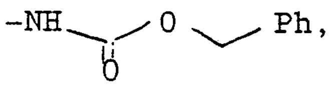

xiii) $-NR_3^{\oplus}A^{\ominus}$ wherein $A^{\ominus}$ is a counterion,
xiv) $-NR^{15}R^{16}$ wherein $R^{15}$ and $R^{16}$ are the same or different and are $C_{1-5}$ alkyl joined together directly to form a 5-7 membered heterocycle,
xv) aryl,
xvi) -CHO,
xvii) $-OP(O)(OR_x)_2$ wherein $R_x$ is H or aryl, or
xviii)
$-O-\overset{O}{\overset{\|}{C}}-C_{1-4}$ alkyl substituted with one or more of amine or quaternary amine;
c) $-(CH_2CH_2O)_nCH_3$ or $-(CH_2CH_2O)_nH$;
2) $-N(R^{14})_2$,
3) $-NR^{15}R^{16}$ wherein $R^{15}$ and $R^{16}$ are defined above, or
4)

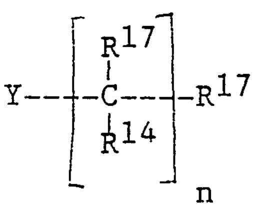

wherein:
Y, $R^{14}$, and n are defined above, and
$R^{17}$ is
a) hydrogen;
b) aryl unsubstituted or substituted with one or more of
i) halo,
ii) -OR, wherein R is H or $C_{1-4}$ alkyl,
iii)
$-\overset{O}{\overset{\|}{C}}OR$,
iv)
$-\overset{O}{\overset{\|}{C}}NR_2$,
v) $-CH_2NR_2$,
vi) $-SO_2NR_2$,
vii) $-NR^2_2$,
viii)

$-NH\overset{O}{\overset{\|}{C}}R$,
ix) $C_{1-4}$ alkyl,
x) phenyl,
xi) $-CF_3$,
xii)
$-\overset{R}{\overset{|}{N}}-SO_2R$,
xiii) $-C_{1-4}$ alkyl-$NR_2$,
xiv) $-OP(O)(OR_x)_2$ wherein $R_x$ is H or aryl, or
xv)
$-O-\overset{O}{\overset{\|}{C}}-C_{1-4}$ alkyl substituted with one or more of amine or quaternary amine;
c) heterocycle, unsubstituted or substituted with one or more of
i) halo,
ii) -OR, wherein R is H, $C_{1-4}$ alkyl, or $C_{1-4}$ alkenyl,
iii)
$-\overset{O}{\overset{\|}{C}}OR$,
iv)
$-\overset{O}{\overset{\|}{C}}NR_2$,
v) $-CH_2NR_2$,
vi) $-SO_2NR_2$,
vii) $-NR_2$,
viii)
$-NH\overset{O}{\overset{\|}{C}}R$,
ix) $C_{1-4}$ alkyl,
x) phenyl,
xi) $-CF_3$,
xii)
$-\overset{R}{\overset{|}{N}}-SO_2R$,
xiii) phenyl $C_{1-4}$ alkyl,
xiv)
$-O\overset{O}{\overset{\|}{C}}R$,
xv) $-OP(O)(OR_x)_2$ wherein $R_x$ is H or aryl, or
xvi)
$-O-\overset{O}{\overset{\|}{C}}-C_{1-4}$ alkyl substituted with one or more of amine or quaternary amine;
d) A 5- to 7-membered carbocyclic or 7- to 10-membered bicyclic carbocyclic ring which is either saturated or unsaturated, such as cyclopentane, cyclohexane, indan, norbornane or naphthalene, the carbocyclic ring being unsubstituted or substituted with one or more of
i) halo,
ii) -OR, wherein R is H or $C_{1-4}$ alkyl,
iii)
$-\overset{O}{\overset{\|}{C}}OR$,
iv)
$-\overset{O}{\overset{\|}{C}}NR_2$,
v) $-CH_2NR_2$,
vi) $-SO_2NR_2$,
vii) $-NR_2$,
viii)
$-NH\overset{O}{\overset{\|}{C}}R$,
ix) $C_{1-4}$ alkyl,
x) phenyl,
xi) $-CF_3$,
xii)
$-\overset{R}{\overset{|}{N}}-SO_2R$,
xiii) $-OP(O)(OR_x)_2$ wherein $R_x$ is H or aryl, or
xiv)

$-O-\overset{O}{\overset{\|}{C}}-C_{1-4}$ alkyl substituted with one or more of amine or quaternary amine;
or pharmaceutically acceptable salts.

In the compounds of the present invention, the A, G, B and J components and the like may have asymmetric centers and occur as racemates, racemic mixtures and as individual diastereomers, with all isomeric forms being included in the present invention.

When any variable (e.g., aryl, heterocycle, R, $R^1$, $R^3$, $R^4$, $R^5$, $R^9$, $R^{10}$, $R^{11}$, $R^{12}$, $R^{14}$, $R^{15}$, $R^{16}$, $R^{17}$, $A^-$, n, Y, Z, etc.) occurs more than one time in any constituent or in formula I, its definition on each occurrence is independent of its definition at every other occurrence. Combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; "carboxy" is -COOH; and "cycloalkyl" is intended to include saturated ring groups, such as cyclopropyl, cyclobutyl, cyclopentyl, cyclohexyl (Cyh) and cycloheptyl; "alkenyl" is intended to include hydrocarbon chains of either a straight or branched configuration and one or more unsaturated carbon-carbon bonds which may occur in any stable point along the chain, such as ethenyl, propenyl, butenyl, pentenyl, and the like. "Halo", as used herein, means fluoro, chloro, bromo and iodo; and "counterion" is used to represent a small, single negatively-charged species, such as chloride, bromide, hydroxide, acetate, trifluoroacetate, perchlorate, nitrate, benzoate, maleate, tartrate, hemitartrate, benzene sulfonate, and the like.

As used herein, with exceptions as noted, "aryl" is intended to mean phenyl (Ph) or naphthyl. "Carbocyclic" is intended to mean any stable 5- to 7-membered carbon ring or 7- to 10-membered bicyclic carbon ring, any of which may be saturated or partially unsaturated.

The term heterocycle, as used herein except where noted, represents a stable 5- to 7-membered mono- or bicyclic or stable 7- to 10-membered bicyclic heterocyclic ring which is either saturated or unsaturated, and which consists of carbon atoms and from one to three heteroatoms selected from the group consisting of N, O and S, and wherein the nitrogen, carbon or sulfur atoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, indanyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazolyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl.

One embodiment of the compounds of the present invention encompasses those compounds of Formula I in which B is independently present twice and Z is 0. In this embodiment, it is preferred that J is $NH_2$ and Q is

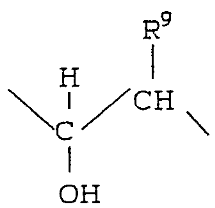 ; 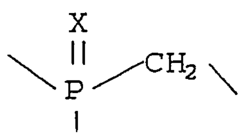 ; or 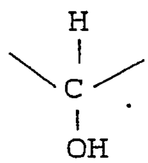 .

A second embodiment of the compounds of the present invention consists of those compounds of Formula I in which B is present once and Z is 0. In this embodiment, it is preferred that Q is

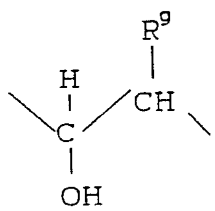 ; 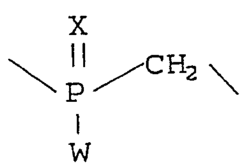 ; or 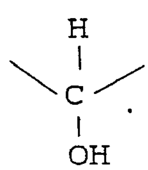 .

A third embodiment of the compounds of the present invention encompasses those compounds of Formula I in which B is absent. In this embodiment, it is preferred that Q is

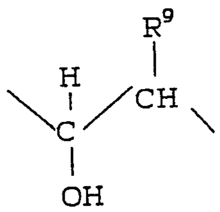 ; 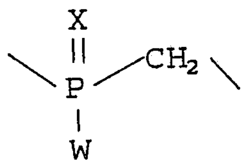 ; or 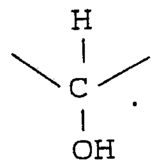 .

A fourth embodiment of the compounds of the present invention encompasses those compounds of Formula I in which G is

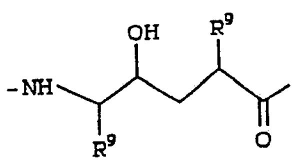 or 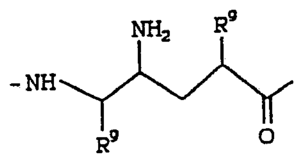 .

A fifth embodiment of the compounds of the present invention encompasses those compounds of formula I in which G is

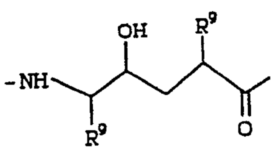 or 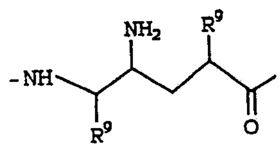 ,

and B is absent or present once.

A sixth embodiment of the compounds of the present invention encompasses those compounds of Formula I in which G is

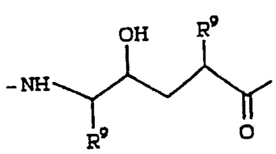 or 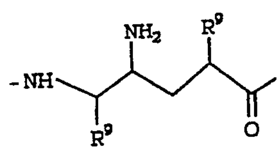 ;

B is absent or present once; and

J is 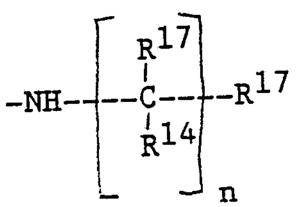 .

A seventh embodiment of the compounds of the present invention encompasses those compounds of Formula I in which A is

$R^1\text{-}\overset{O}{\overset{\|}{C}}\text{-}$ or $R\text{-}SO_2\text{-}$ with the proviso that $R^1$ is not aryl when attached to S; G is

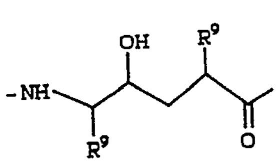 or 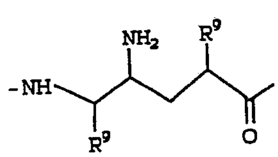 ;

B is absent or present once; and
J is

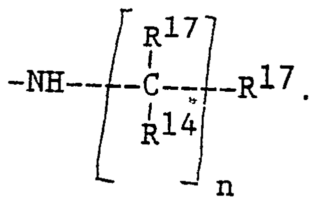

Preferred compounds of the present invention are compounds A,B,C,D, or E, as follows:

A:

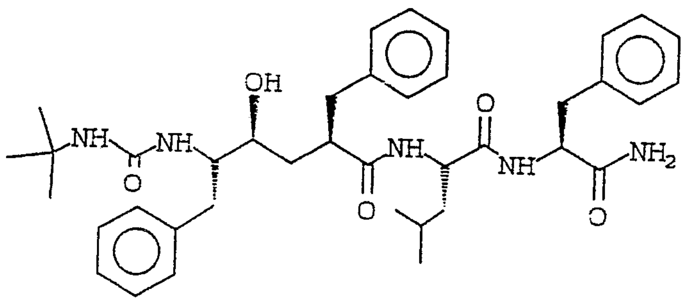

N'-(1,1-dimethylethylaminocarbonyl)-5(S)-amino-4(S)-hydroxy-6-phenyl-2(R)-(phenylmethyl)hexanoyl-leucylphenylalanylamide;

B:

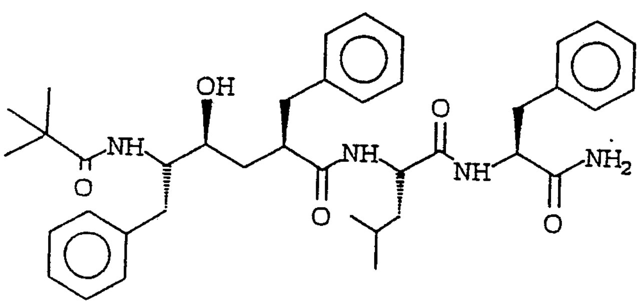

N'-(2,2-dimethylpropanoyl)-5(S)-amino-4(S)-hydroxy-6-phenyl-2(R)-(phenylmethyl)hexanoyl-leucyl-phenylalanylamide;

C:

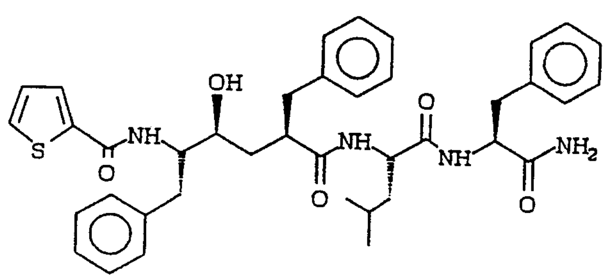

N'-(2-thienoyl)-5(S)-amino-4(S)-hydroxy-6-phenyl-2(R)-(phenylmethyl)hexanoyl-leucyl-phenylalanylamide;

D:

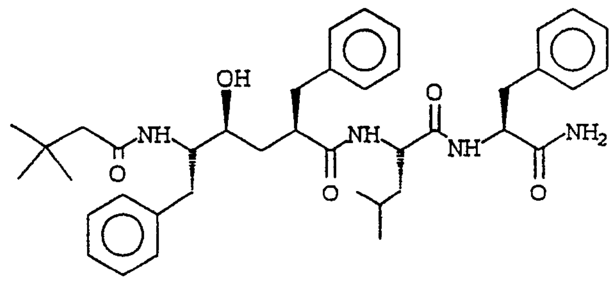

N'-(3,3-dimethylbutanoyl)-5(S)-amino-4(S)-hydroxy-6-phenyl-2(R)-(phenylmethyl)hexanoyl-leucyl-phenylalanylamide;

E:

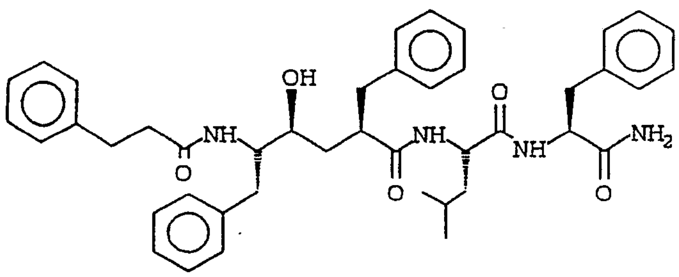

N'-(3-phenylpropanoyl)-5(S)-amino-4(S)-hydroxy-6-phenyl-2(R)-(phenylmethyl)hexanoyl-leucyl-phenylalanylamide.

Other preferred compounds of the present invention include compounds F, G, H, and J, as follows:

F:

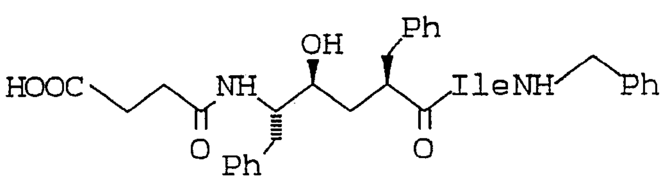

N-benzyl-N'-(succinoyl)-5(S)-amino-4(S)-hydroxy-6-phenyl-2(R)-(phenylmethyl)hexanoyl isoleucyl amide;

G:

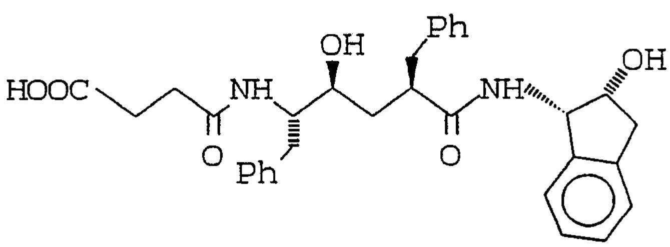

N-(2(R)-hydroxy-1(S)-indanyl)-N'-(succinoyl)-5(S)-amino-4(S)-hydroxy-6-phenyl-2(R)-(phenylmethyl)-hexanamide;

H:

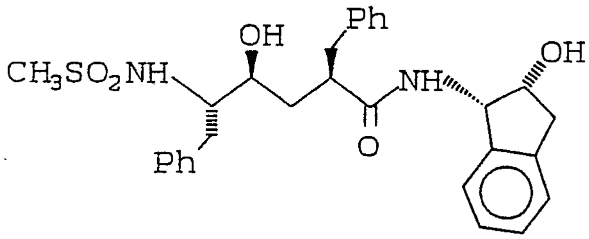

N-(2(R)-hydroxy-1(S)-indanyl)-N′-(methanesulfonyl)-5(S)-amino-4(S)-hydroxy-6-phenyl-2(R)-(phenylmethyl)-hexanamide;

J:

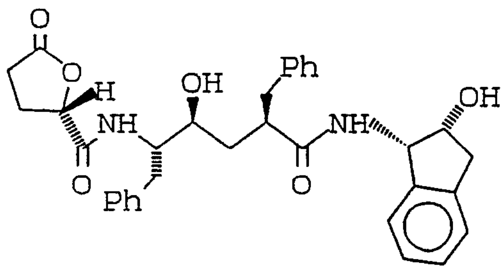

N-(2(R)-hydroxy-1(S)-indanyl)-N′-(5-oxo-2(S)-tetrahydrofuranylcarbonyl)-5(S)-amino-4(S)-hydroxy-6-phenyl-2(R)-(phenylmethyl)hexanamide, or pharmaceutically acceptable salts thereof.

Other preferred compounds include the following:

N′-(2,2-dimethylpropanoyl)-5(S)-amino-4(S)-hydroxy-6-phenyl-2(R)-(phenylmethyl)-hexanoyl -Leu-Phe-amide,

N′-(1,1-dimethylethylaminocarbonyl)-5(S)-amino-4(S)-hydroxy-2(R)-phenylmethyl-hexanoyl-Leucyl-Phenylalanyl-amide,

N′-(3-phenylpropanoyl)-5(S)-amino)-4(S)-hydroxy-6-phenyl-2(R)-(phenylmethyl)-hexanoyl-Leucyl-Phenylalanyl-amide,

N′-succinoyl-5(S)-amino-4(S)-hydroxy-6-phenyl-2(R)-(phenylmethyl)hexanoyl-N-(phenylmethyl)-Ile-amide,

N-(2(R)-hydroxy-1(S)-indanyl)-N′-(4-t-butyldimethylsilyloxybutano)-5(S)-amino-4(S)-hydroxy-6-phenyl-2(R)-(phenylmethyl)hexanamide,

N-(2(R)-hydroxy-1(S)-indanyl)-N′-(methanesulfonyl)-5(S)-amino-4(S)-hydroxy-6-phenyl-2(R)-(phenylmethyl)-hexanamide,

N-(2(R)-hydroxy-1(S)-indanyl)-N′-(4-hydroxybutanoyl)-5(S)-amino-4(S)-hydroxy-6-phenyl-2(R)-phenylmethyl-hexanamide,

N-(2(R)-hydroxy-1(S)-indanyl)-N′-[2-(2-[2-methoxyethoxy]ethoxy)ethoxycarbonyl]-5(S)-amino-2(R)-benzyl-4(S)-hydroxy-6-phenylhexanamide,

N′-(methanesulfonyl)-5(S)-amino-4(S)-hydroxy-6-phenyl-2(R)-(phenylmethyl)hexanoyl-N-[2,3-dihydroxypropyl]-Val amide,

or pharmaceutically acceptable salts thereof.

The pharmaceutically-acceptable salt or salts of the compounds of Formula I (in the form of water- or oil-soluble or dispersible products) include the conventional non-toxic salts or the quaternary ammonium salts of these compounds, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

HIV protease inhibitors of Formula I may be prepared in accordance with well-known procedures for

preparing peptide analogs from their constituent amino acids or analogs thereof.

In general, once the G substituent is made, the rest of the synthesis follows the principle and practice of amide bond formation by the coupling methods of either solution-phase or solid-phase peptide synthesis. The addition and removal of one or more protecting groups is also typical practice.

Structures and abbreviations for the G components of the inhibitors of the present invention include:

1. 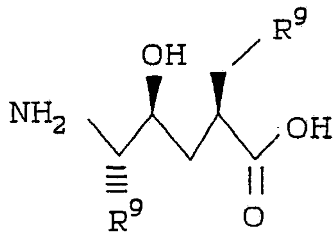

,

a hydroxyethylene dipeptide isostere prepared, for example, by an intermediate lactone according to Evans, B.E. et al J. Org. Chem. 50, 4615(1985) or Kempf, D.J. J. Org. Chem. 51, 3921(1986). Synthetic routes to similar peptide bond isosteres, such as

2. 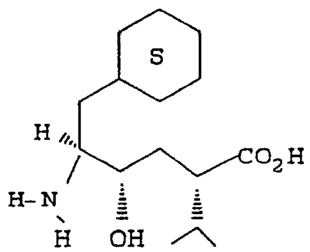

Cal[CH(OH)$CH_2$]Val,

are readily available. The intermediate BOC-Cal[CH(OH)$CH_2$]Val-OH lactone is obtained from intermediates prepared using methods described by P. Buhlmayer et al, in Published European Patent Application 184,550-A2. Other synthetic routes to peptide bond isosteres of like character are described in the following:

a. Szelke et al, in Peptides, Structure and Function. Proceedings of the Eighth American Peptide Symp(ed. V.J. Hruby and D.H. Rich) pp. 579-82, Pierce Chemical Co., Rockford, IL;

b. D.T. Pals et al in European Patent Appln. 173,481-A2;

c. A.H. Fray et al, J. Org. Chem., 51, 4828-4833 (1986); and

d. M. Szelke et al, PCT Int. Appl. WO 84 03,044;

Structures and abbreviations for other G components of the inhibitors of the present invention include:

3. 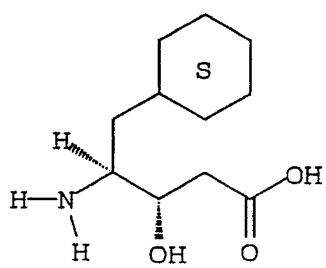

ACHPA

with BOC-ACHPA-OEt being prepared by the method described by Boger et al., J. Med. Chem., 28, 1779-1790 (1985);

4. 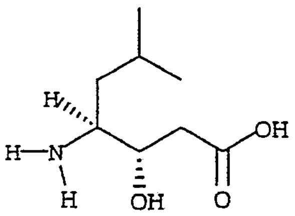

Statine (Sta)

with BOC-Sta-OEt being prepared in accordance with the procedure described by Rich et al., J. Org. Chem., 43, 3624 (1978);

5. 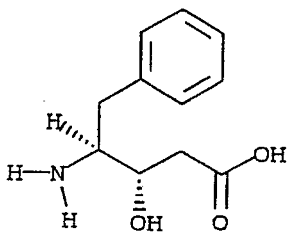

AHPPA

with BOC-AHPPA-OEt being prepared as described by Rich et al., J. Org. Chem., 23, 27-33 (1980);

6. 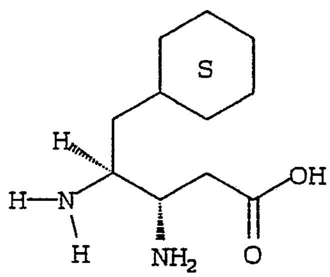

AmACHPA

7. 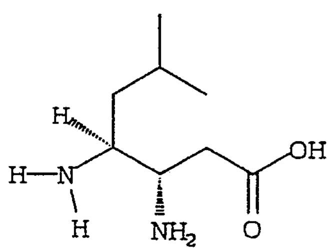

AmSta

with BOC-AmSta(CBZ)-OH being prepared as shown in the following Scheme, and BOC-AmACHPA(CBZ)-OH being prepared as illustrated in the Scheme by substituting BOC-ACHPA-OEt for BOC-Sta-OEt:

Synthesis of protected 3-amino-3-deoxy-(3S, 4S)-statine:

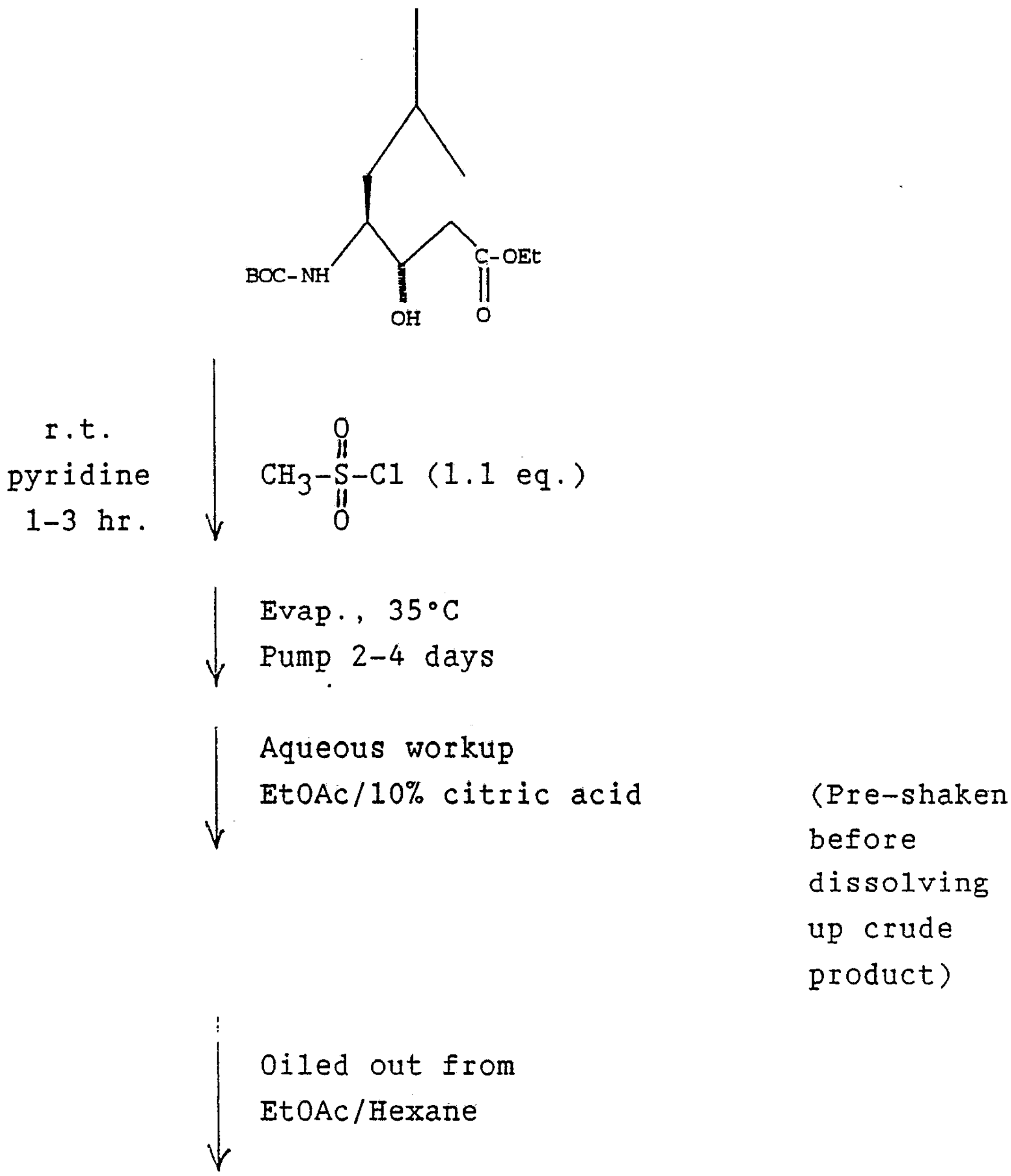

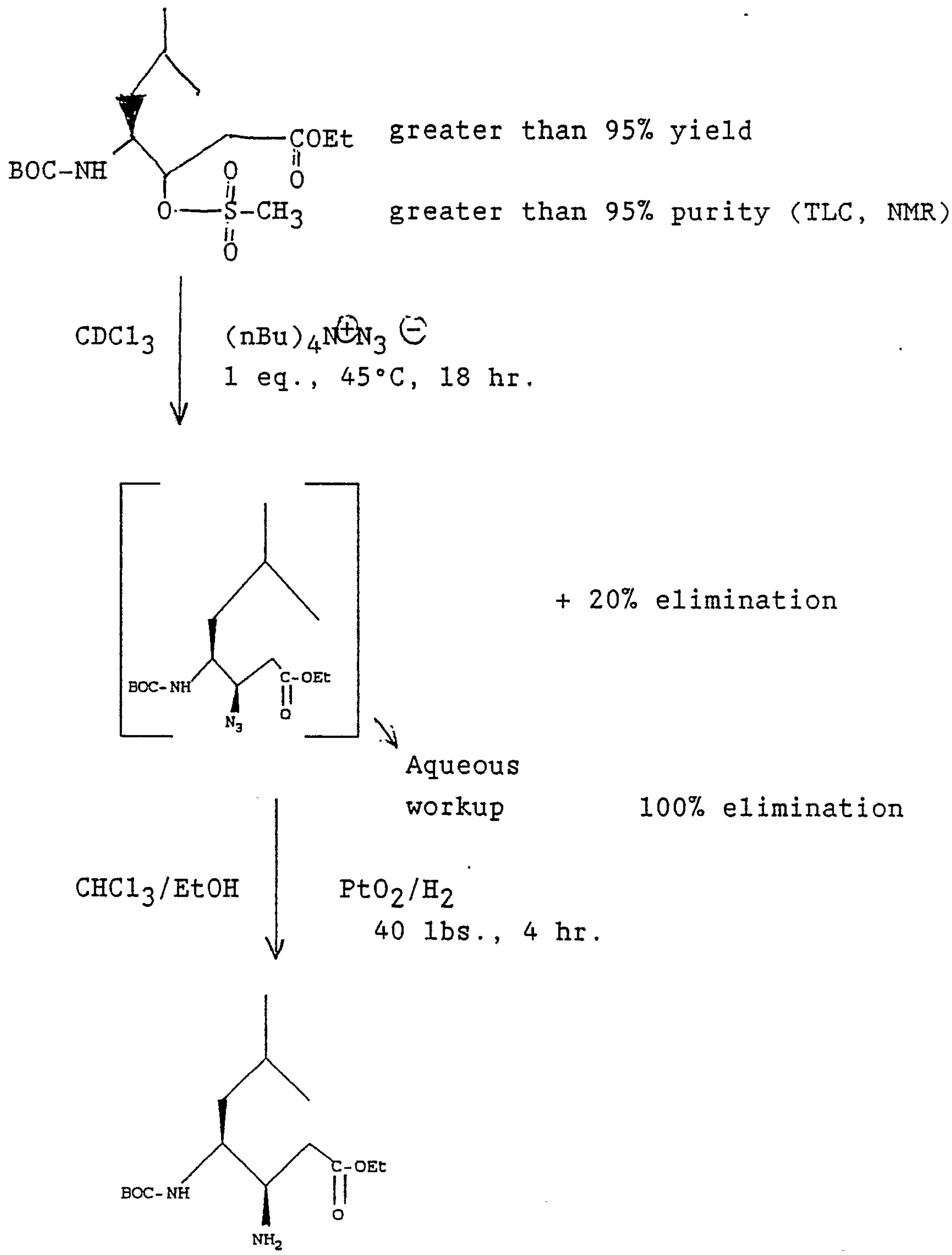
greater than 95% yield
greater than 95% purity (TLC, NMR)
CDCl3
(nBu)4N⊕N3⊖
1 eq., 45°C, 18 hr.
+ 20% elimination
Aqueous
workup
100% elimination
CHCl3/EtOH
PtO2/H2
40 lbs., 4 hr.

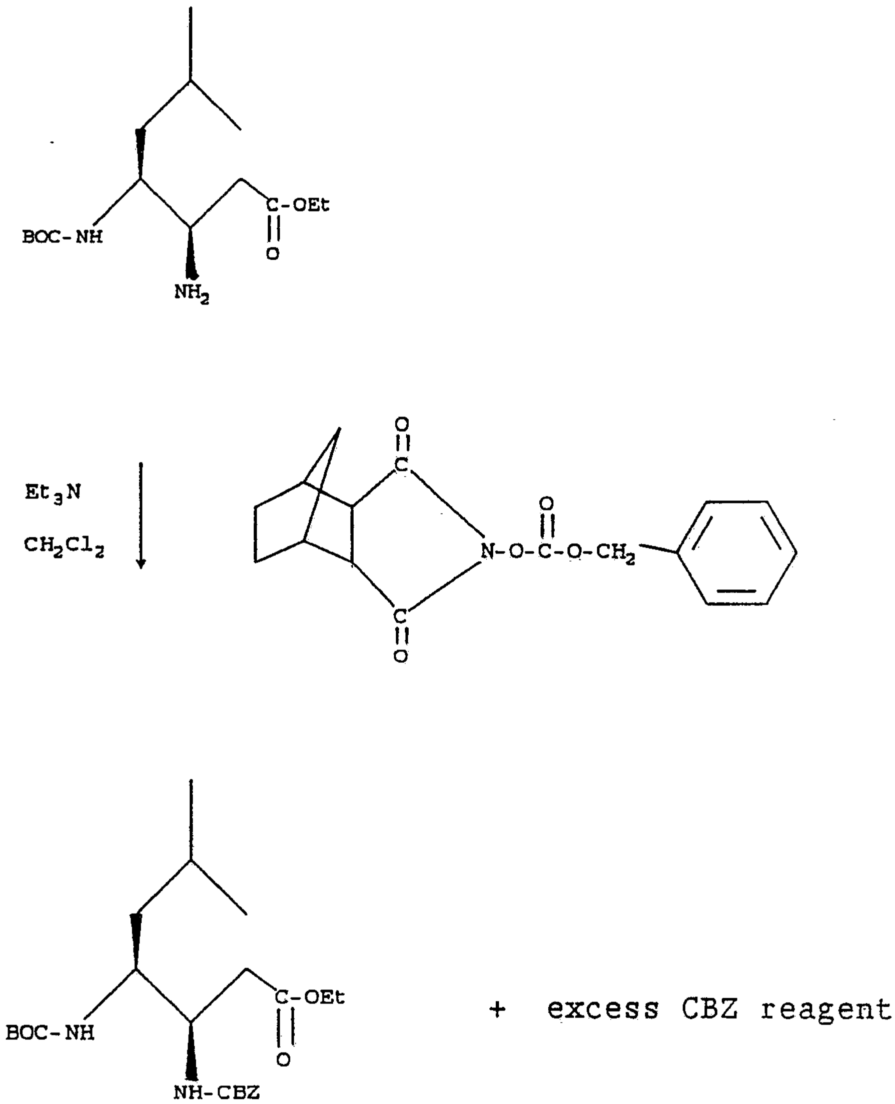


(predominantly R at position 3 as shown).

Base hydrolysis gives the free acid for incorporation into the synthesis of the compounds of Formula I; or alternatively, this material may be prepared as described by Jones et al, in Peptides, Structure and Function. Proceedings of the Ninth American Peptide Symposium (eds. C. M. Deber, V. J. Hruby and K. D. Kopple) pp. 759-62, 1985, Pierce Chemical Co., Rockford, IL. ; Arrowsmith et al, J. Chem. Soc. Chem. Commun. 755-7 (1986); and Raddatz et al, Published Eur. Pat. Appl. 161,588; with efficient methods for preparing the 2-substituted statine component G (such as

8. 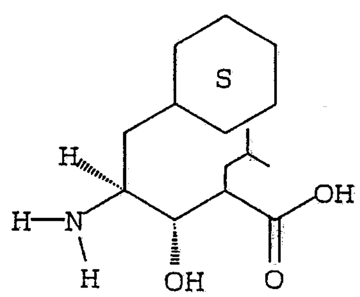

(2-isobutyl)ACHPA

in a suitably protected form being described in Pub. Eur. Pat Appln. 157,409 (with other pertinent references including D. Veber et al, Biochem. Soc. Trans., 12, 956-959 (1984) and H. Stein et al, Fed. Proc. 45, 869, Abstract No 4151 (1986).

Amide couplings used to form the compounds of this invention are typically performed by the carbodiimide method with reagents such as dicyclohexylcarbodiimide, or N-ethyl, N′-(3-dimethylaminopropyl) carbodiimide. Other methods of forming the amide or peptide bond include, but are not limited to synthetic routes via an acid chloride, azide, mixed anhydride or activated ester. Typically, solution phase amide couplings with or without peptide fragments are performed, but solid-phase synthesis by classical Merrifield techniques may be employed instead.

The selection of protecting groups is, in part, dictated by particular coupling conditions, and in part by the amino acid and peptide components involved in the reaction. The amino-protecting groups ordinarily employed include those which are well-known in the art, for example, urethane protecting substituents such as benzyloxycarbonyl (carbobenzoxy), p-methoxycarbobenzoxy, p-nitrocarbobenzoxy, t-butyloxycarbonyl, and the like. It is preferred to utilize t-butyloxycarbonyl (BOC) for protecting the α-amino group, in part because the BOC protecting group is readily removed by relatively mild acids such as trifluoroacetic acid (TFA), or hydrogen chloride in ethyl acetate. By these procedures the A group may be added to deprotected G-B-B-J compounds of Formula I.

The OH group of Thr and Ser may be protected by the Bzl (benzyl) group and the ε-amino group of Lys may be protected by the IPOC group or the 2-chlorobenzyloxycarbonyl (2-Cl-CBZ) group. Treatment with HF or catalytic hydrogenation are typically employed for removal of IPOC or 2-Cl-CBZ.

One scheme for preparing compounds of formula I is presented below. Example 1 specifically illustrates the application of the following scheme (I) to specific compounds.

## SCHEME I

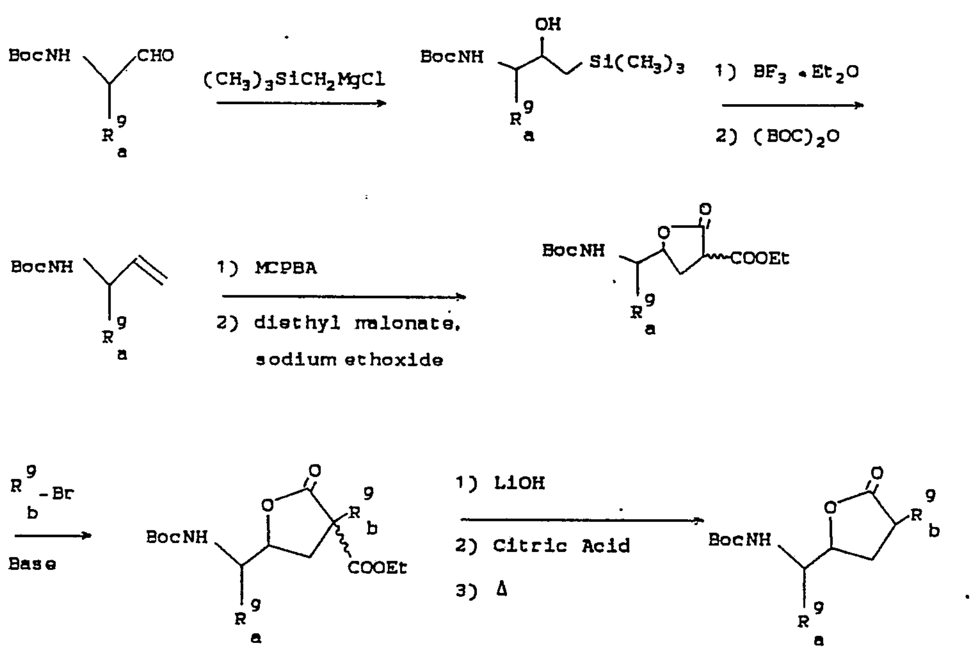


Illustrations of the product of Scheme I include the following compounds of Table I.

TABLE I

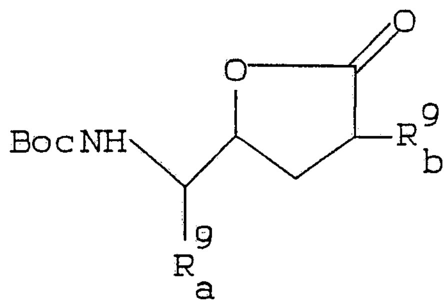

| $R^9_a$ | $R^9_b$ |
| --- | --- |
| $PhCH_2-$ | $-H$ |
| $PhCH_2-$ | $-(CH_2)_3CH_3$ |
| $PhCH_2-$ | $-CH_2$ 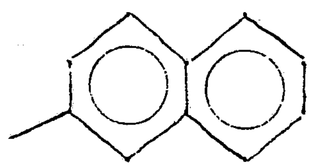 |
| $PhCH_2-$ | $-CH_2$ 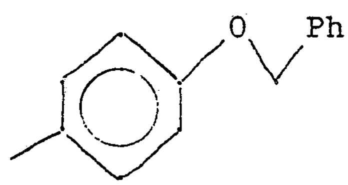 |
| $PhCH_2-$ | $-CH_2$ 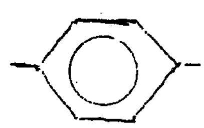 $-O(CH_2)_2Ph$ |

| $R^9_a$ | $R^9_b$ |
|---|---|
| $PhCH_2-$ | $-CH_3$ |
| $PhCH_2-$ | $-CH_2-CH=CH-$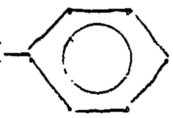 |
| $PhCH_2-$ | $-CH_2-$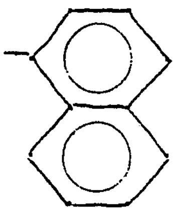 |
| $PhCH_2-$ | $-(CH_2)_2OCH_3$ |
| $PhCH_2-$ | $-CH_2-$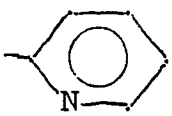 |
| 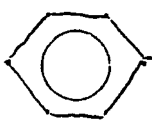$-CH_2-$ | $-CH_2Ph$ |
| $CH_3(CH_2)_3-$ | $-CH_2PH$ |
| $PhCH_2O-$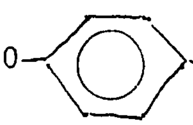$-CH_2-$ | $-CH_2Ph$ |

| $R^9_a$ | $R^9_b$ |
|---|---|
| 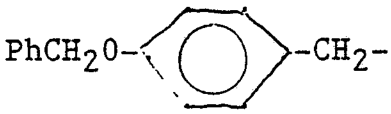 | 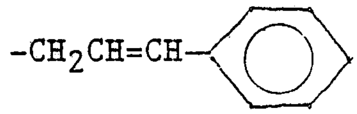 |
| $PhCH_2OCH_2-$ | $-CH_2Ph$ |
| 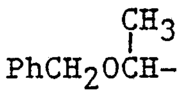 | 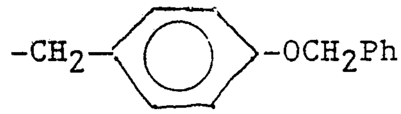 |
| $(CH_3)_2CHCH_2-$ | $-CH_2CH(CH_3)_2$ |
| $PhCH_2-$ | 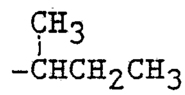 |
| $(CH_3)_2CH-$ | $-CH_2Ph$ |
| 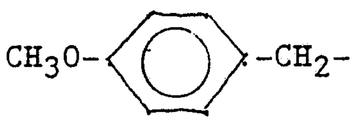 | $-CH_2Ph$ |
| 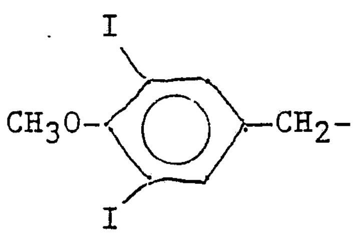 | $-CH_2Ph$ |
| $PhCH_2-$ | 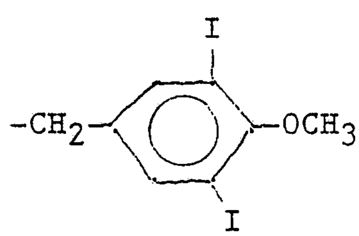 |

| $R^9_a$ | $R^9_b$ |
|---|---|
| $PhCH_2-$ | $-(CH_2)_3Ph$ |
| $PhCH_2-$ | 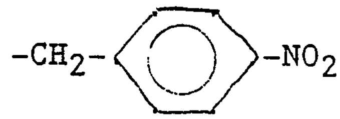 |
| $PhCH_2-$ | 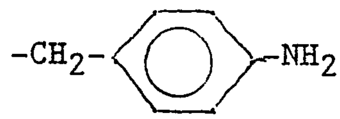 |
| $PhCH_2-$ | 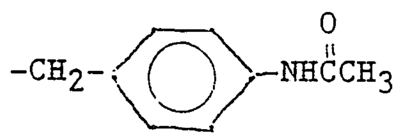 |
| 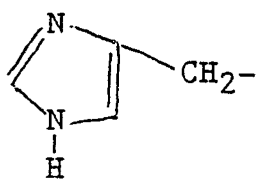 | $-CH_2Ph$ |
| $PhCH_2-$ | 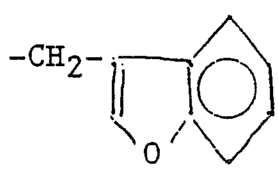 |
| $PhCH_2-$ | 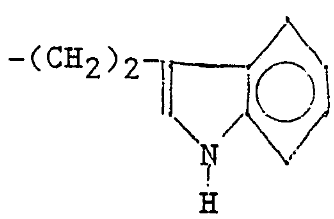 |

| $R^{9}{}_{\underline{a}}$ | $R^{9}{}_{\underline{b}}$ |
|---|---|
| 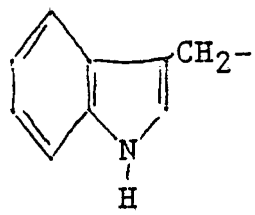 | 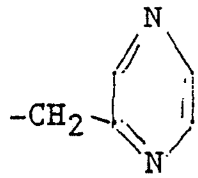 |
| $PhCH_2-$ | $-CH_2SPh$ |
| $PhCH_2-$ | 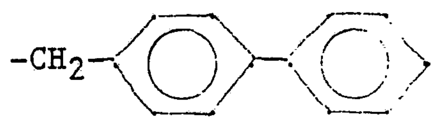 |
| $PhCH_2-$ | $CH_3$ |
| $PhCH_2-$ | $-CH_2CH_3$. |

B groups are added onto the carboxyl terminal end by, for example, amide coupling in Scheme II. See Example I for specific illustration.

SCHEME II

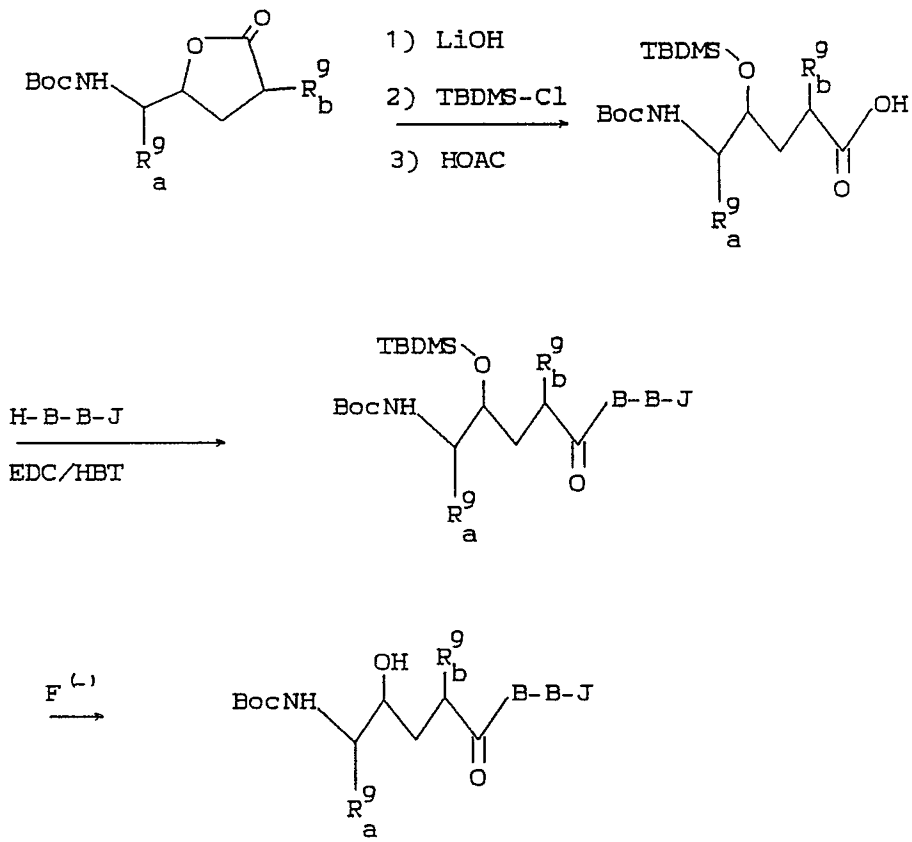


Products of Scheme II include compounds with substituents listed in Table I.

TABLE II

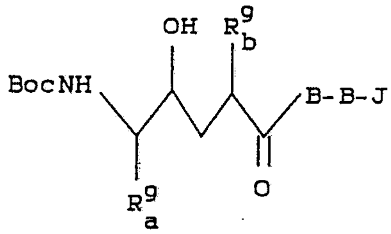

| $R^9_a$ | $R^9_b$ | B-B-J |
|---|---|---|
| $PhCH_2$- | $-CH_2-CH=CH-Ph$ | -LeuPhe[$NH_2$] |
| 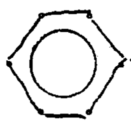$-CH_2-$ | $-CH_2Ph$ | -LeuPhe[$NH_2$] |
| $PhCH_2$- | $-CH_2Ph$ | -Ile[$NH_2$] |
| $PhCH_2$- | $-CH_2Ph$ | -LeuNH 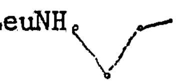 $NHSO_2CH_3$ |
| $PhCH_2$- | $-CH_2Ph$ | -LeuPhe[$OCH_3$] |
| $PhCH_2$- | $-(CH_2)_3CH_3$ | -LeuPhe[$NH_2$] |
| $PhCH_2$- | $-CH_2Ph$ | -IleNH 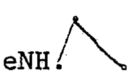 Ph |

| $R^9a$ | $R^9b$ | B-B-J |
|---|---|---|
| $PhCH_2-$ | $-CH_2-$ 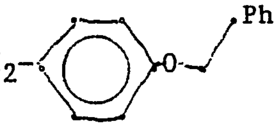 | -LeuPhe[$NH_2$] |
| $CH_3(CH_2)_3-$ | $-CH_2Ph$ | -LeuPhe[$NH_2$] |
| $PhCH_2-$ | $-CH_2Ph$ | -LeuNH 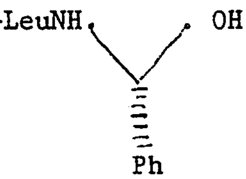 |
| $PhCH_2$ | $-CH_2Ph$ | -ValNH 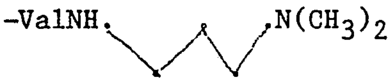 |
| $PhCH_2-$ | $-CH_2$ 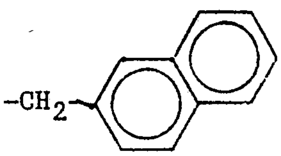 | -LeuPhe[$NH_2$] |
| $PhCh_2-$ | $-CH_2Ph$ | -LeuNH 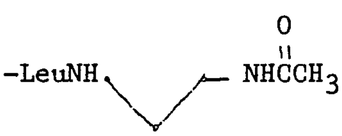 |
| $PhCH_2-$ | $-CH_2Ph$ | -LeuNH 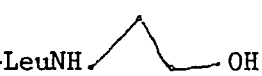 |
| $PhCH_2-$ | $-CH_2Ph$ | -LeuPhe |
| $PhCH_2-$ | $-CH_2Ph$ | -NH- 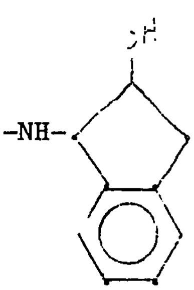 |

| $R^9_a$ | $R^9_b$ | B-B-J |
|---|---|---|
| 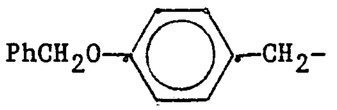 | $-CH_2Ph$ | 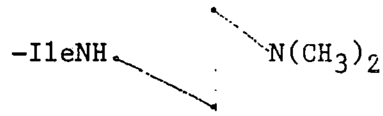 |
| $PhCH_2O-CH_2-$ | $-CH_2-CH_2-CH_2-Ph$ | $-IlePhe[NH_2]$ |
| 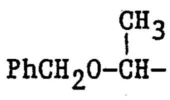 | $-CH_2Ph$ | 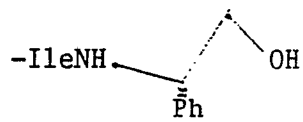 |
| $PhCH_2-$ | $-CH_2Ph$ | $-(CH_2)_2NHSO_2CH_3$ |
| $PhCH_2-$ | $-CH_2-CH=CH-Ph$ | 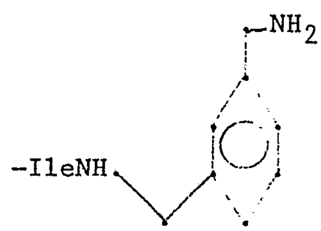 |
| $PhCH_2-$ | $-CH_2Ph$ | 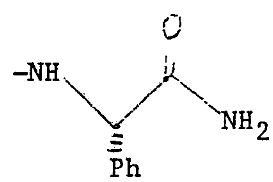 |

Removal of the Boc protecting group is followed by preparation of the acylated amines according to Scheme III, which is specifically illustrated by Example 1.

## SCHEME III

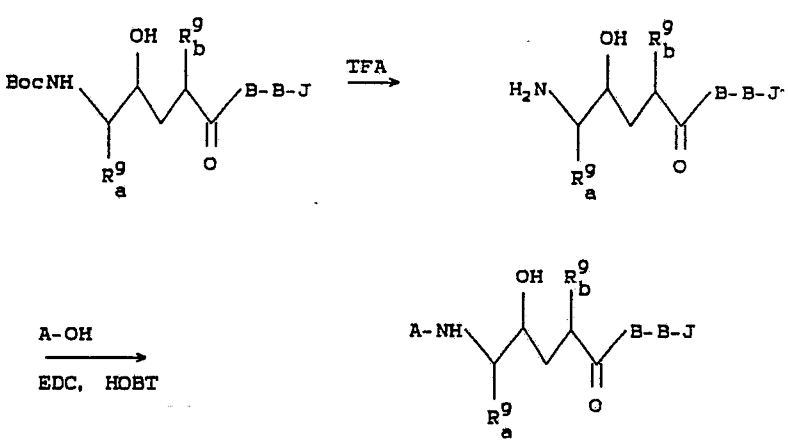

Product compounds of scheme III include those of Table III.

## TABLE III

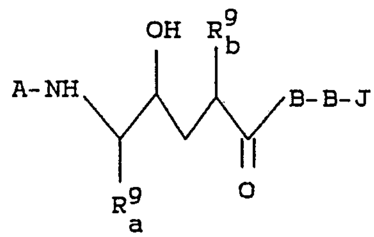

| A | $R^9_a$ | $R^9_b$ | B-B-J |
|---|---|---|---|
| $(CH_3)_3CCH_2\overset{O}{\overset{\|}{C}}-$ | $PhCH_2-$ | $-CH_2Ph$ | $-LeuPhe[NH_2]$ |
| $(CH_3)_3C\overset{O}{\overset{\|}{C}}-$ | 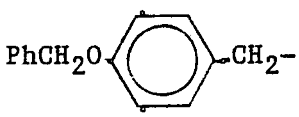 | $-CH_2Ph$ | 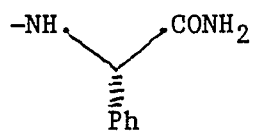 |
| 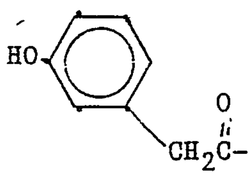 | $PhCH_2-$ | $-CH_2Ph$ | $-NHCH_2Ph$ |
| 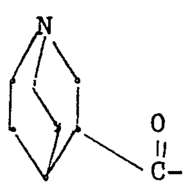 | $(CH_3)_2CCH_2-$ | $-CH_2CH{=}CHPh$ | $-GluPhe[NH_2]$ |

Other end groups of the amino terminal side are added by reaction of the amine with the desired acid chloride, as an electrophile, in the reaction in Scheme IV:

SCHEME IV

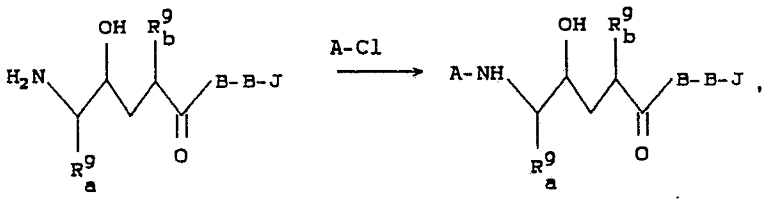


wherein A is defined in formula I. See also Example 2. Product compounds of Scheme IV include those of Table IV.

TABLE IV

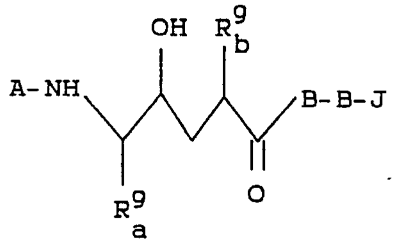

| A | $R^9_a$ | $R^9_b$ | B-B-J |
|---|---|---|---|
| $CH_3SO_2-$ | $PhCH_2-$ | $-CH_2Ph$ | -Leu Phe $NH_2$ |
| $(CH_3)_3CNHSO_2-$ | $PhCH_2-$ | $-CH_2CH=CHPh$ | 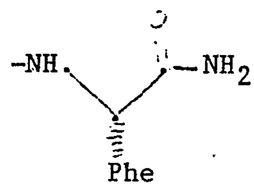 |
| 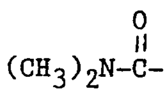 | 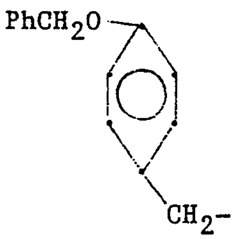 | $-CH_2Ph$ | -Ile$[NH_2]$ |
| 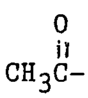 | 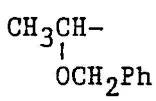 | 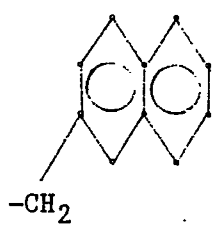 | $-NHCH_2Ph$ |

| | | | |
|---|---|---|---|
| 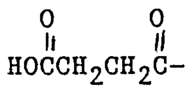 | $PhCH_2-$ | $-CH_2Ph$ | 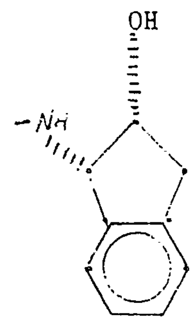 |
| 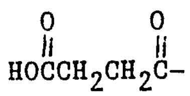 | $PhCH_2-$ | $-CH_2Ph$ | 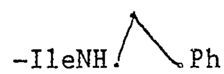 |

Additional J groups are added by Schemes Va and Vb:

Va: 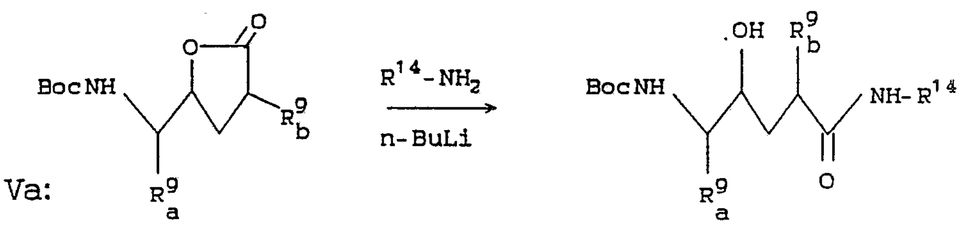


Vb: 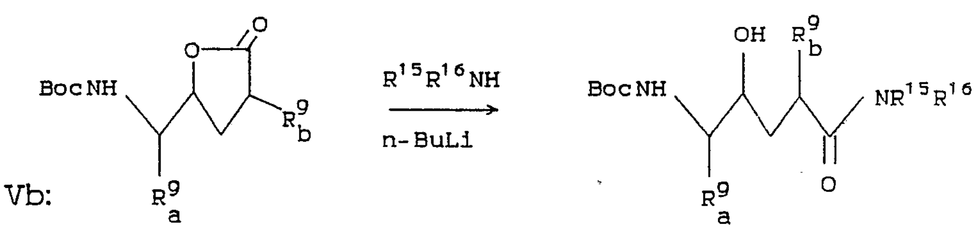


Similarly with other J groups under the definition of Formula I. See also Example 4. Product compounds include those of the following Tables.

TABLE Va

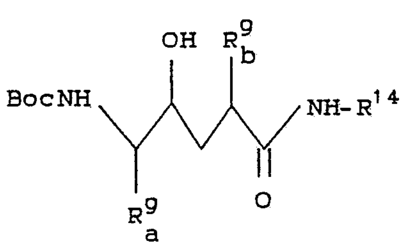

| $R^9_a$ | $R^9_b$ | $NH-R^{14}$ and other J groups. |
|---|---|---|
| $PhCH_2-$ | $-CH_2Ph$ | $-NH-(CH_2)_2CH(CH_3)_2$ |
| $PhCH_2-$ | $-CH_2Ph$ | 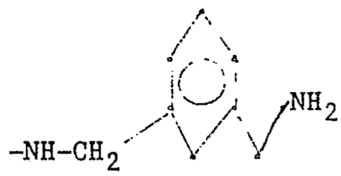 |
| $PhCH_2-$ | $-CH_2Ph$ | 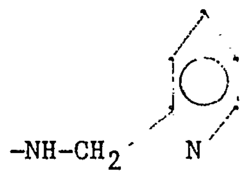 |
| $PhCH_2-$ | $-CH_2Ph$ | 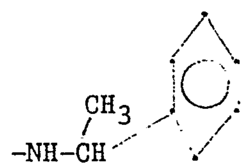 |
| $PhCH_2-$ | $-CH_2Ph$ | 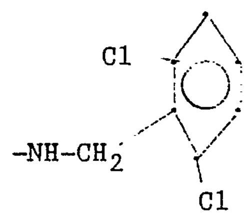 |

TABLE Vb

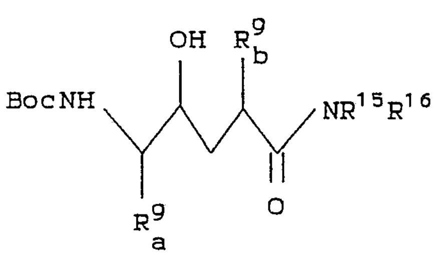

| $R^9{}_a$ | $R^9{}_b$ | $NR^{15}R^{16}$ |
|---|---|---|
| $PhCH_2-$ | $-CH_2Ph$ | 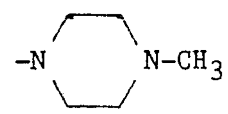 |
| $PhCH_2-$ | $-CH_2Ph$ | 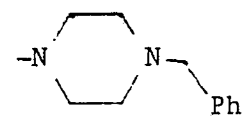 |
| $PhCH_2-$ | $-CH_2Ph$ | 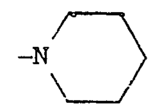 |
| $PhCH_2-$ | $-CH_2Ph$ | 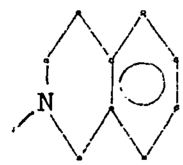 a benzopiperdinyl J group. |
| $PhCH_2-$ | $-CH_2Ph$ | 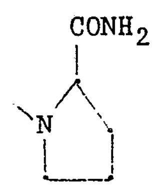 |

The compounds of the present invention are useful in the inhibition of HIV protease, the prevention or treatment of infection by the human immunodeficiency virus (HIV), and the treatment of consequent pathological conditions such as AIDS. Treating AIDS, preventing infection by HIV or treating infection by HIV is defined as including, but not limited to, treating a wide range of states of HIV infection: AIDS, ARC (AIDS related complex), both symptomatic and asymtomatic, and actual or potential exposure to HIV. For example, the compounds of this invention are useful in preventing infection by HIV after suspected past exposure to HIV by e.g., blood transfusion, accidental needle stick, or exposure to patient blood during surgery.

In the present invention, compounds with asymmetric centers may occur as racemates, racemic mixtures and as individual diastereomers, with all isomeric forms of the compounds being included in the present invention.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination the severity of the particular condition, and the host undergoing therapy.

The present invention is also directed to combinations of the HIV protease-inhibitory compounds with one or more agents useful in the treatment of AIDS.

For example, the compounds of this invention can be given in combination with the antivirals, immunomodulaters, antibiotics or vaccines or other derivative forms thereof as listed in the Table VI [source: Marketletter, Nov. 30, 1987, pp. 26-27; Genetic Engineering News, Jan. 1988, Vol. 8, 23]:

TABLE VI[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| FOSCARNET (trisodium phosphonoformate) | Astra AB | HIV inf, CMV retinitis |

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| RETROVIR (zidovudine; AZT) | Burroughs Wellcome | AIDS, advanced ARC<br>pediatric AIDS, KS, asympt HIV, less severe HIV, neurological in-volvement. |
| RIFABUTIN (ansamycin LM 427) | Adria Labs | ARC |
| (trimetrexate) | Warner-Lambert | PCP |
| UA001 | Ueno Fine Chem Industry | AIDS, ARC |

| Drug Name | Manufacturer | Indication |
| --- | --- | --- |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| | | |
| --- | --- | --- |
| ABPP AIDS, KS (bropirimine) | Upjohn | Advanced |
| AMPLIGEN (mismatched RNA) | DuPont HEM Research | ARC, PGL |
| (Anti-human alpha interferon antibody) | Advanced Biotherapy Concepts | AIDS, ARC, KS |
| Colony Stimulating HIV, Factor (GM-CSF) | Sandoz Genetics Institute | AIDS, ARC, KS |
| CL246,738 (CL246,738) | American Cynamid | AIDS |

| Drug Name | Manufacturer | Indication |
|---|---|---|
| IMREG-1 | Imreg | AIDS, ARC, |
| PGL, | | KS |
| IMREG-2 | Imreg | AIDS, ARC, |
| PGL, | | KS |
| IMUTHIOL (diethyl dithio carbamate) | Merieux Institute | AIDS, ARC |
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |
| MTP-PE (muramyl-tripep-tide) | Ciba-Geigy | KS |

| Drug Name | Manufacturer | Indication |
|---|---|---|
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |

C. Antibiotics

| | | |
|---|---|---|
| PENTAM 300 (pentamidine isethionate) | LyphoMed | PCP |

D. Vaccines

Any one of a variety of AIDS or HIV vaccines presently under study and development can be used in combination with the compounds of this invention or salt or derivative forms thereof, in the treatment or prevention of AIDS and diseases of similar character caused by HIV.

It will be understood that the scope of combinations of the compounds of this invention with AIDS antivirals, immunomodulators, antibiotics or vaccines is not limited to the list in the above Table, but includes in principle any combination with any pharmaceutical composition useful for the treatment of AIDS.

SYNTHESIS

The preparation and synthesis follows, in general, U.S. Patent 4,661,473; Evans, B.E. et al, J. Org. Chem., 50, 4615, (1985); Evans, B.E. et al., "A Stereocontrolled Synthesis of Hydroxyethylene Dipeptide Isosteres," Proc. Am. Pept. Symp., 9, 743-6(198), and Luly, J.R. et al., J. Org. Chem 52, 1487 (1987), all herein incorporated by reference.

EXAMPLE 1

Preparation of N′-(2-thienocarbonyl)-5(S)-amino-4(S)-hydroxy-6-phenyl-2(R)-(phenylmethyl)-hexanoyl-Leucyl-phenylalanyl amide, Compound C

Step A: Preparation of 3(S)-[(1,1-Dimethylethoxycarbonyl)amino]-2(RS)-hydroxy-4-phenyl-1-trimethylsilylbutane

To a stirred suspension of magnesium turnings (9.79 g, 403 mmol) in dry diethyl ether (200 ml) under nitrogen was added chloromethyltrimethylsilane (50 ml, 358 mmol). The reaction was initiated by gentle warming and then was cooled in an ice bath to maintain gentle reflux. After the exotherm was complete the reaction was stirred at room temperature for 1 hour then cooled to 78°C in a dry ice/acetone bath. To the solution of the Grignard was added dropwise with stirring a solution of N-2(S)-[(1,1-dimethylethoxycarbonyl)amino]-3-phenyl propionaldehyde (19.3 g, 77.4 mmol) in dry diethyl ether (250 ml) dropwise such that the temperature of the reaction remained below -55°C. The resultant gray suspension was allowed to warm to room temperature where it was stirred for 30 minutes then was quenched by pouring into a mixture of ice (500 g) and 10% citric acid (500 ml). The organic phase was collected and the aqueous phase was extracted with diethyl ether (3X300 ml). The combined organics were washed with 10% citric acid (1X300 ml) and brine (1X200 ml), dried over anhydrous magnesium sulfate, filtered, and concentrated to give crude 3(S)-[(1,1-dimethylethoxycarbonyl)amino]-2(RS)-hydroxy-4-phenyl-1-trimethylsilyl butane (26.6 g, quantitative crude yield) as a yellow oil, an analytical sample was obtained by low pressure chromatography (silica gel, 230-400 mesh; diethyl ether:hexanes, 30%:70%) followed by recrystallization from heptane. mp = 91-95°C;

elemental analysis, calcd. for $C_{18}H_{31}NO_3$ Si (337.53):

| | | | | |
|---|---|---|---|---|
| | C, 64.05; | H, 9.26; | N, 4.15; | |
| Found: | C, 64.15; | H, 9.13; | N, 4.22; | $[\alpha]_D^{20}$ = -40.0° |

Step B: Preparation of 3(S)-Amino-4-phenyl-1-butene

To a stirred solution of the product of Step A (22,8 g, 67.5 mmol) in dry methylene chloride (400 ml) cooled in an ice bath and under nitrogen was added in a fine stream boron trifluoride etherate (43 ml, 345 mmol). The solution was allowed to warm to room temperature where it was stirred for 4 days. Reaction was cooled in an ice bath and quenched by the dropwise addition to 10% sodium hydroxide (400 ml). The organic phase was collected and the aqueous phase was extracted with methylene chloride (2X250 ml). The combined organics were washed with brine (1X200 ml), dried over anhydrous magnesium sulfate, filtered, and concentrated to give crude 3(S)-amino-4-phenyl-1-butene (14.2 g) as a yellow oil.

Step C: Preparation of N-3(S)-[(1,1-Dimethylethoxycarbonyl)amino]-4-phenyl-1-butene

A solution of the product of Step B (14.2 g) and di-tert-butyl dicarbonate (31.0g, 142 mmol) in dry methylene chloride (200 ml) was stirred at room temperature for 18 hours., washed with 10% citric acid (3X100 ml), water (1X100 ml), sat'd. sodium bicarbonate (3X125 ml), and brine (1X250 ml), dried over anhydrous magnesium sulfate, filtered, and concentrated to yield crude N-3(S)-[(1,1-dimethylethoxycarbonyl)amino]-4-phenylbutene (34.6 g) as a yellow oil. Crude product was purified by low pressure chromatography (silica gel. 230-400 mesh, 10x20 cm column; diethyl ether:hexanes, 20%:80%) to yield N-3(S)-[(1,1-dimethylethoxycarbonyl)amino]-4-phenyl-1-butene (16.3 g, 97.6% yield) as a white solid. An analytical sample was obtained by recrystallization from heptane, mp = 67.5-68.5°C;

elemental analysis, calcd. for $C_{15}H_{21}NO_2$ (247.34):

| | | | |
|---|---|---|---|
| | C, 72.84; | H, 8.56; | N, 5.66; |
| Found: | C, 72.78; | H, 8.76; | N, 5.64. |

Step D: Preparation of 1(R)-[1′(S)-(1,1-Dimethylethoxycarbonyl)amino-2-phenylethyl]oxirane

To a solution of the product of Step C (9.4 g, 38 mmol) in dry methylene chloride (100 ml) cooled in an ice bath and under nitrogen was added 3-chloroperoxybenzoic acid (technical grade, 80-85%; 41 g, ~200 mmol). The mixture was stirred at 0°C for 18 hours and at 25°C for 23 hours., then diluted with diethyl ether (300 ml), and poured in ice cold aq. 10% sodium sulfite (1 L). The organic layer was collected and the aqueous layer was extracted with diethyl ether (2X100 ml). The combined organics were washed with 10% sodium sulfate (3 x 100 ml), sat'd. sodium bicarbonate (3X100 ml), and brine (1x100 ml), dried over anhyd. sodium sulfate, filtered and concentrated to give a white solid. Crude product was purified by low pressure chromatography (silica gel 230-400 mesh, 8 X 15 cm column; ethyl acetate:hexanes, 25%:75%) to yield 1(R)-[1′(S)-(1,1-dimethylethoxycarbonyl)amino-2-phenylethyl]oxirane (7.0 g, 70% yield) as a clear oil which crystallized upon standing. An analytical sample was obtained by recrystallization from heptane. mp = 51.5-52°C;

elemental analysis, calcd. for $C_{15}H_{21}NO_3$ (263.34):

| | | | | |
|---|---|---|---|---|
| | C, 68.42; | H, 8.04; | N, 5.32; | |
| Found: | C, 68.22; | H, 8.26; | N, 5.29; | $[\alpha]_D^{20}$ = -1.34°. |

Step E: Preparation of (5S, 1'S)-3-carboethoxy-5-(1-(1,1-dimethylethoxycarbonyl)amino)-2-phenylethyl)-dihydrofuran-2-(3H)-one

The product from Step D, 9.93 g, was dissolved in 100 mL of absolute ethanol and added to a solution of 2.6g of sodium and 20.1 mL of diethyl malonate in 170 mL of absolute ethanol. After stirring overnight, the reaction was acidified to pH ~ 4 with 10% citric acid and extracted with 2X 500 mL of ether. The combined organic extracts were washed 1X500 mL $H_2O$, 1X500 mL sat'd $NaHCO_3$, 1X500 mL sat'd brine and dried over $MgSO_4$. The solvents were removed and the crude product purified by low pressure chromatography on silica gel eluting with 50% ether/hexanes (or EtOAc/hexane). The yield of semi-solid product was 10.6g. The later fractions contained 2.5 g of the undesired 5R isomer as a white solid.

Step F: Preparation of (5S, 1'S)-3-carboethoxy-3-phenylmethyl-5-(1-((1,1-dimethylethoxycarbonyl)amino)-2-phenylethyl)dihydrofuran--2-(3H)-one

The product of Step E, 10.6 g, was dissolved in 100 mL of abs. ethanol containing 3.7 mL of benzyl bromide and added to a solution of 0.71 g of sodium in 100 mL of absolute ethanol. The solution was heated to 50° C for 1.5 hours, then cooled in an ice bath and acidified with 500 mL of 10% citric acid. The mixture was extracted 3X500 mL of ether and the combined ether extracts washed with 400 mL of $H_2O$, 400 mL of brine, dried ($MgSO_4$) and the solvent removed under reduced pressure to give 13.6 g of a clear colorless oil which was essentially homogeneous by TLC (25% ethyl acetate/hexanes).

Step G: Preparation of (3R, 5S, 1'S)-3-Benzyl-5-(1-((1,1-dimethylethoxycarbonyl)amino)-2-phenylethyl)dihydrofuran-2-(3H)-one

The product of Step F, 13.6 g, was dissolved in 250 mL of 1,2-dimethoxy ethane, and to it was added 117 mL of 1M lithium hydroxide at room temperature. After stirring for 12 hours, the solvents were removed under reduced pressure, the residue suspended in 200 mL of 10% citric acid and extracted 3X 500 mL of diethyl ether. The combined ether extract were washed with 500 mL of brine, dried ($MgSO_4$) and concentrated to dryness. The residue was dissolved in 250 mL of toluene, heated to reflux for 12 hours, then concentrated to dryness under reduced pressure. Purification by medium pressure chromatography over silica gel, eluting with 15% ethyl acetate/hexanes gave 3.2 g of the 3R-lactone as a clear foam. Further elution with the same solvents gave 6.15 g of the 3S-lactone as a white solid.

Step H: Preparation of N'-(1,1-dimethylethoxycarbonyl)-5(S)-amino-4(S)-(1',1'-dimethylethyl-1,1-dimethylsilyloxy)-6-phenyl-2(R)-(phenylmethyl)-hexanoic acid

(3R, 5S, 1'S)-3-Benzyl-5-(1-((1,1-dimethylethoxycarbonyl)amino)-2-phenylethyl)dihydrofuran-2-(3H)-one, 0.5g, was dissolved in 30 mL of a 2:1 mixture of ethylene glycol dimethyl ether/water, and to it was added 5 mL of 1M lithium hydroxide at room temperature. After stirring for 1 hour, the solvent was removed in vacuo and the residue partitioned between 20 mL chloroform and 20 mL 10% citric acid. The layers were separated and the aqueous phase extracted with 3 X 20 mL chloroform. The combined organic layers were dried ($Na_2SO_4$) and the solvent removed to yield 0.46 g of the crude hydroxy acid. This residue was dissolved in 5 mL of dry DMF and 0.845 g tert-butyl dimethylsilyl chloride and 0.725 g of imidazole were added. After stirring for 18 hours, the reaction was poured into 50 mL of water and extracted with 3 X 20 mL of ethyl acetate. The combined organic extracts were washed with 3 X 20 mL of 10% citric acid, 1 X 20 mL of water, 3 X 10 mL of saturated aqueous solution of $Na_2CO_3$, and 20 mL of brine. After drying ($Na_2SO_4$), the solvent was removed and the resulting residue dissolved in a mixture of 5 mL of THF, 5 mL of glacial acetic acid, and 2 mL of water. The mixture was stirred for 4 hours, then poured into 50 mL of water and extracted with 3 X 20 mL of ether. The combined ether extracts were washed with 2 X 20 mL of water, brine, dried ($Na_2SO_4$), and the solvent removed. Purification by medium pressure chromatography over silica gel, eluting with $MeOH/CHCl_3$ gave 0.53 g of the product as a white solid.

Step I: Preparation of N'-(1,1-dimethylethoxycarbonyl)-5(S)-amino-4(S)-(1',1'-dimethylethyl-1,1-dimethylsilyloxy)-6-phenyl-2(R)-(phenylmethyl)-hexanoyleucyl-phenylalanyl amide.

The product from Step H, 0.183 g, was dissolved in 10 mL of dry DMF, and to it was added 0.124 g of leucinyl-phenylalanyl amide hydrochloride hemihydrate, 0.051 g of 1-hydroxybenzotriazole hydrate and 0.069 g of 1-ethyl-3-(3-dimethylaminopropyl) carbodiimide hydrochloride. Triethylamine was added to the stirring solution until the pH was 8.5. After stirring for 2 hours, the reaction was poured into 80 mL of water and extracted with 5 X 10 mL of ethyl acetate. The combined organic extracts were washed with 3 X 20 mL of 10% citric acid, 1 X 20 mL of water, 3 X 20 mL of a saturated aqueous solution of $Na_2CO_3$, 30 mL of brine, dried

($Na_2SO_4$), and the solvent removed to give 0.2 g of the product after purification by preparative thin layer chromatography (5% methanol/chloroform).

Step J: Preparation of N'-(1,1-dimethylethoxycarbonyl)-5(S)-amino-4(S)-hydroxy-6-phenyl-2(R)-(phenylmethyl)hexanoyl-leucyl-phenylalanyl amide

The product from Step I, 0.2 g, was placed in a flask and to it was added 2 mL of a 1M solution of tetrabutylammonium fluoride in THF. After stirring for 2 hours, the solvent was removed in vacuo, and the residue was taken up in 50 mL of 10% methanol/chloroform and passed through a pad of silica gel. The solvent was removed in vacuo, and the remaining solid triturated with ethyl acetate. The solid was filtered through a silica pad and washed with ethyl acetate. The silica gel pad was then washed with chloroform (200 mL). The chloroform solution of the product was evaporated to yield 0.074 g of pure product. The ethyl acetate solution was concentrated, and the residue was chromatographed over silica gel, eluting with methanol/chloroform to give an additional 0.054 g of product. The combined yield of the product was 0.128 g: mp 218-210°C.

Step K: The Preparation of 5(S)-amino-4-(S)-hydroxy-6-phenyl-2(R)-(phenylmethyl)-hexanoyl-Leucyl-Phenyalanyl Amide

To a solution of 350 mgs. (0.49 mmol) of N'-((1,1-dimethylethoxy)carbonyl)-5(S)-amino-4(S)-hydroxy-6-phenyl-2(R)-(phenylmethyl)-hexanoyl-Leucyl-Phenyalanyl Amide dissolved in 15 mls. of methylene chloride at 0°C was added 5 mls. of trifluoroacetic acid. After 30 minutes, a thin layer chromatogram (TLC) showed that the reaction was complete. The reaction solution was concentrated, the residue dissolved in 25 mls of methylene chloride and transfered to a separatory funnel. The organic layer was washed with 2x50 ml of sat'd. $NaHCO_3$ solution and the organic layer was dried ($Na_2SO_4$), filtered and concentrated to give the crude product which was pure enough to be used in the next reaction.

Step L: The preparation of N'-(2-thienocarbonyl)-5(S)-amino-4(S)-hydroxy-6-phenyl-2(R)-(phenylmethyl)-hexanoyl-Leucyl-Phenyalanyl Amide

To a solution of 30 mgs. (0.049 mmol) of 5(S)-amino-4(S)-hydroxy-6-phenyl-2(R)-(phenylmethyl)-hexanoyl-Leucyl-Phenyalanyl Amide in 10 mls of degassed dimethyl formamide (DMF) was added 10 mgs (0.074 mmol) of 1-hydroxybenzotriazole hydrate (HOBT), 14 mgs (0.074 mmol) of 1-(3-Dimethylaminopropyl)-3-ethylcarbodiimide hydrochloride (EDC) and 7 mgs (0.055 mmol) of thiophene-2-carboxylic acid. The pH of the solution was adjusted to ca. 9.0 with triethylamine and the reaction was stirred at room temperature. After 18 hours a tlc indicated that the coupling was complete. The DMF was removed in vacuo and the residue dissolved in 20 mls. of methylene chloride which was washed with 2X20 mls of 10% citric acid soln., 20 mls of satd. $NaHCO_3$ solution and the organic layer was dried, filtered and concentrated to give the crude product. The residue was purified using prep. layer chromatography (5% MeOH/$CHCL_3$, $SiO_2$, 0.5 mm plate) to give 18 mgs of the title compound m.p. 232-234°C.

## EXAMPLE 2

Preparation of N'-(3-phenylpropanoyl)-5(S)-amino-4(S)-hydroxy-6-phenyl-2(R)-(phenylmethyl)-hexanoyl-Leucyl-Phenylalanyl amide, Compound E

To a solution of 0.038 mmol of 5(S)-amino-4(S)-2-(R)-(phenylmethyl)-hexanoyl-Leucyl-Phenyalanyl Amide in 10 mls of methylene chloride which contained 10 µl (0.072 mmol) of triethylamine is added 10 ul (0.070 mmol) of 3-phenylpropanoyl chloride. After 3 hours the reaction is poured into a separatory funnel and washed with 2X10 mls of 10% citric acid solution, 10 mls of satd. $NaHCO_3$ solution. The organic phase is dried, filtered, and concentrated to dryness. The concentrate is passed through a pad of $SiO_2$ which is eluted with 5% MeOH/$CHCL_3$ to give the crude product. This is further purified using reverse phase HPLC on a Water C-18 column to give the desired product.

## EXAMPLE 3

Preparation of N'-(1,1-Dimethylethylcarbonyl)-5(S)-amino-4(S)-hydroxy-6-phenyl-2(R)-(4-hydroxyphenylmethyl)hexanoyl-leucyl-phenylalanyl amide

Step A: Preparation of N'-(1,1-Dimethylethoxycarbonyl)-5(S)-amino-4(S)-hydroxy-6-phenyl-2(R)-(4-hydroxyphenylmethyl)hexanoyl-leucyl-phenylalanyl amide

To a stirred solution of N′-(1,1-dimethylethoxycarbonyl)-5(S)-amino-4(S)-hydroxy-6-phenyl-2(R)-(4-benzyloxyphenylmethyl)hexanoyl-leucyl-phenylalanyl amide, 25 mg, in 10 mL of tetrahydrofuran was added a suspension of 10% palladium of carbon, 25 mg, in 10 mL of abs. methanol. The mixture was stirred under an atmosphere of hydrogen for 4 hours at room temperature, then filtered and concentrated to dryness. The residue was dissolved in 1 mL of tetrahydrofuran and 1 mL of water was added. A white solid precipitated which was collected and dried under vacuum over $P_2O_5$. The yield was 20 mg of pure product. mp 222-223° C. (C,H,N)

Step B: Preparation of 5(S)-amino-4(S)-hydroxy-6-phenyl-2(R)-(4-hydroxyphenylmethyl)hexanoyl-leucyl-phenylalanyl amide

In a manner substantially similar to that described in Step K of Example 1, N′-(1,1-dimethylethoxycarbonyl)-5(S)-amino-4(S)-hydroxy-6-phenyl-2(R)-(4-hydroxyphenylmethyl)hexanoyl-leucyl-phenylalanyl amide is converted to the named product.

Step C: Preparation of N′-(1,1-dimethylethylcarbonyl)-5(S)-amino-4(S)-hydroxy-6-phenyl-2(R)-(4-hydroxyphenylmethyl)hexanoyl-leucyl-phenylalanyl amide

Following substantially the procedure described in Step L of Example 1, but employing the amine from Step B of this Example and pivalic acid, the described product is obtained.

## EXAMPLE 4

Preparation of N-benzyl-N′-(4-hydroxyphenylmethylcarbonyl)-5(S)-amino-4(S)-hydroxy-6-phenyl-2(R)-(phenylmethyl)hexanoic carboxamide

Step A: Preparation of N-benzyl-N′-(1,1-dimethylethoxycarbonyl)-5(S)-amino-4(S)-hydroxy-6-phenyl-2(R)-(phenylmethyl)hexanoic carboxamide

The lithium salt of benzyl amine was prepared by adding 0.5 mL of a 2.5M solution of n-butyl lithium to 0.134 g of benzyl amine in 10 mL of dry THF at -78° C. To this was added 0.1 g of (3R, 5S, 1′S)-3-benzyl-5-(1-((1,1-dimethylethoxycarbonyl)amino)-2-phenylethyl)dihydrofuran-2-(3H)- one in 5 mL of THF. After stirring for 15 minutes, the reaction was quenched by the addition of 15 mL of 10% citric acid, and the mixture was allowed to warm to room temperature. The volume was tripled by the addition of ethyl acetate. The organic layer was separated, and the aqueous phase was washed with ethyl acetate. The combined organic solutions were washed with 3 x 10 mL 10% citric acid, 1 x 20 mL of saturated $Na_2CO_3$ solution, brine, and dried ($Na_2SO_4$). The solvent was removed in vacuo to yield a white solid. The solid was recrystallized from hexane/ethyl acetate to yield 0.065 g of the product as a crystalline solid; mp 191-192° C.

Step B: Preparation of N-benzyl-5(S)-amino-4(S)-hydroxy-6-phenyl-2(R)-(phenylmethyl)hexanoic carboxamide

The amine protecting group is removed by following substantially the procedure described in Step K of Example 1 yielding desired product.

Step C: Preparation of N-benzyl-N′-hydroxyphenylmethylcarbonyl)-5(S)-amino-4(S)-hydroxy-6-phenyl-2(R)-(phenylmethyl)hexanoic carboxamide

A new amine protecting group is added to the product of Step B of this Example, by following substantially the procedure described in Step L of Example 1, but replacing the 2-thiophenecarboxylic acid used therein, and substituting thereto 4-hydroxyphenylacetic acid. The indicated product is obtained.

## EXAMPLE 5

Preparation of N′-(1,1-dimethylethylaminosulfonyl)-4(S)-amino-3(S)-hydroxy-5-cyclohexylpentanoyl-leucyl-phenylalanyl amide

Step A: Preparation of N-(1,1-dimethylethoxycarbonyl)-4(S)-amino-3(S)-hydroxy-5-cyclohexylpentanoyl-leucyl-phenylalanyl amide

N-(1,1-dimethylethoxycarbonyl)-4(S)-amino-3(S)-hydroxy-5-cyclohexylpentanoic acid (Boc-ACHPA), (0.575g, 1.82 mmol) was dissolved in 5.5 mL of dry DMF, under argon. To this well stirred mixture was added

L-leucyl-phenylalanine amide hydrochloride semihydrate (0.74g, 2.18 mmol), hydroxybenztriazole hydrate (0.258g, 1.91 mmol), ethyl 3-(dimethylamino)propylcarbodiimide hydrochloride (0.374g, 1.91 mmol), and triethylamine (0.57 mL, 4.10 mmol). This mixture was stirred at room temperature for 1 hour. The reaction mixture was diluted with ethyl acetate (30 mL). This mixture was washed with water (3X15 mL), 10% aqueous citric acid (15 mL), and brine (15 mL). Drying ($Na_2SO_4$), filtration, and removal of the solvent in vacuo gave the crude coupling product. This material was chromatographed on silica gel using 6% methanol in chloroform as eluant. There was obtained 0.582 g of the title compound as a white crystalline solid. MP: 171-175° C.

Step B: Preparation of 4(S)-amino-3(S)-hydroxy-5-cyclohexylpentanoyl-leucyl-phenylalanyl amide

The amine protecting group is removed by following substantially the procedure described in Step K of Example 1, yielding desired product.

Step C: Preparation of N'-(1,1-dimethylethylaminosulfonyl)-4(S)-amino-3(S)-hydroxy-5-cyclohexylpentanoyl-leucyl-phenylalanyl amide.

A new amine protecting group is added to the product of Step B in this Example by following substantially the procedure described in Example 2, but replacing the 3-phenylpropanoyl chloride used therein and substituting thereto 1,1-dimethylethyl aminosulfonyl chloride. The indicated product is obtained.

## EXAMPLE 6

Preparation of N'-[3-[(1(R)-Dimethylaminocarbonylamino-2-phenylethyl)phosphinyl]-2(R)-phenylmethyl propanoyl]-leucyl-phenylalanylamide, and N'-[[3-(1(R)-Benzyloxycarbonylamino-2-phenylethyl)phosphinyl]-2(S)-phenylmethyl propanoyl]-leucyl-phenylalanylamide

Step A: Preparation of Methyl-1(R)-benzyloxycarbonylamino-2-phenylethylphosphinate

A 10% solution of trimethylsilyldiazomethane in pentane was added dropwise to a solution of 0.790 g of 1(R)-benzyloxycarbonylamino-2-phenylethyl phosphonous acid in a mixture of benzene (35 mL)/methanol (5 mL) until a pale yellow color persisted in the stirred reaction solution. After one hour at 0° the solution was concentrated and the residue chromatographed over silica gel, eluting with methylene chloride/acetone/methanol (18:1:1), to afford 0.400 g of pure methyl ester.

Step B: Preparation of Methyl-1(R)-benzyloxycarbonylamino-2-phenylethyl-(2(R,S)-carbomethoxy-3-phenyl-1-propyl)phosphinate

The product of Step A, 0.397g, was dissolved in 5 mL of abs. methanol and, at 0°, was treated with 0.67 mL of a 2.0 M solution of sodium methoxide in methanol under a nitrogen atmosphere. After 10 minutes, methyl 2-benzyl acrylate (0.240 g) was added in one portion, the cooling bath removed, and the mixture was stirred at ambient temperature of 18 hours. The mixture was concentrated to an oil which was partitioned between 15 mL of 1N hydrochloric acid and 10 mL of ethyl acetate. The layers were separated and the aqueous phase was extracted with 2X10 mL ethyl acetate. The combined organic layers were washed (brine) and dried ($Na_2SO_4$) and the solvent removed to give an oil which was chromatographed on silica gel (flash). Elution with ethyl acetate afforded 0.264 g of the product.

Step C: Preparation of Methyl-1(R)-benzyloxycarbonylamino-2-phenylethyl-(2(R,S)-carbonyl-3-phenyl-1-propyl)phosphinate

A solution of the product of Step B, 0.264 g, in 1.5 mL of 1,2-dimethoxyethane was treated with 0.56 mL of a 1.0 N aqueous lithium hydroxide solution. After stirring under nitrogen for 4 hours at room temperature, the solvent was removed and 30 mL ice water was added to the residue. The resulting mixture was extracted with 2X10 mL ether/ethyl acetate mixtures, following which the aqueous phase was acidified to pH 4-5 using several drops of 50% acetic acid. The acidic mixture was extracted with 3X15 mL ethyl acetate and then the combined acidic organic layers were washed with water, brine, and dried ($MgSO_4$). Removal of the solvent in vacuo left the product as an oil: 0.153 g.

Step D: Preparation of N-[3-[Methoxy-(1(R)-benzyloxycarbonylamino-2-phenylethyl)phosphinyl]-2-(R,S)-phenylmethyl-propanoyl]-leucyl-phenylalanylamide

The product from Step C, 0.153 g, was dissolved in 4 mL of dry acetonitrile and stirred under nitrogen in an ice bath. To the solution were added 0.119 g of L-leucyl L-phenylalanylamide hydrochloride hemihydrate, 0.155 g of benzotriazol-1-yloxytris(dimethylamino)phosphonium hexafluorophosphate, and triethylamine to pH 8.5. After stirring at 0° for 2 hours the mixture was warmed to room temperature and stirred 3 hours further. Ten mL of dilute aqueous sodium chloride was added and the mixture was extracted with 4X10 mL of ethyl acetate. The combined organic extracts were washed with water, 2N hydrochloric acid, water, saturated aqueous

sodium bicarbonate, brine, and dried ($MgSO_4$). Removal of the solvent left 0.164 g of a sticky solid which was a mixture of 4 major components as evidenced by thin layer chromatography (chloroform/methanol/ammonium hydroxide, 90:10:1). The mixture was flash chromatographed, eluting with the same solvent mixture used for tlc. Early fractions containing an intimate mixture of 3 components were combined and concentrated to give 66 mg of a diastereomeric mixture of the product. Continued elution provided and additional 52 mg of product mixture which was predominately a single tlc spot.

Step E: Preparation of N-[3-[1(R)-Benzyloxycarbonylamino-2-phenylethyl)phosphinyl]-2(R)-phenylmethyl propanoyl]-leucyl-phenylalanylamide, Compound Alpha, and N-[3-[1(R)-Benzyloxycarbonylamino-2-phenylethyl)phosphinyl]-2(S)-phenylmethyl propanoyl]-leucyl-phenylalanyl/amide, Compound Beta

A solution containing 0.066 g of the diastereomeric mixture of Step D and 0.014 g of anhydrous lithium iodide in 2 mL dry tetrahydrofuran was stirred at room temperature for 7 days, during which time an additional 0.030 g of lithium iodide was added. At the end of 1 week the solvent ws removed and the residue suspended in 5 mL of saturated sodium bicarbonate. The mixture was extracted with 4X5 mL ethyl acetate and the combined organic layers were concentrated. Separation of the two diastereomers was accomplished by preparative high pressure liquid chromatography using a Waters Delta Pak C-18 column, eluting with a gradient of acetonitrile, 0-95% in water (0.1% trifluoroacetic acid) over one hour. The fractions of retention time 4.22 minutes and that of 7.00 minutes were lyophilized separately to afford 0.013 g of Compound Alpha and 0.012 g of Compound Beta, respectively.

Step F: Preparation of N-[3-[(1(R)-amino-2-phenylethyl)phosphinyl]-2(R)-phenylmethylpropanoyl]-leucyl-phenylalanyl amide

Following substantially the procedure described in Step A of Example 3, but substituting for the benzyl ether used therein, Compound Alpha from the preceding step, the indicated product is obtained.

Step G: Preparation of N-[3-[(1(R)-dimethylaminocarbonylamino-2-phenylethyl)phosphinyl]-2(R)-phenylmethylpropanoyl]-leucyl-phenylalanyl amide

Following substantially the procedure described in Example 2, but substituting for the amine used therein the product from the preceding step, and for the 3-phenylpropanoyl chloride used therein, dimethylaminocarbonyl chloride, the desired product is obtained.

## EXAMPLE 7

Assay for Inhibition of Synthetic Viral Protease

Inhibition studies of the reaction of the synthetic protease [amino acid residues 69-167 of the pol open reading frame in Ratner, L. et al, Nature, 313, 277 (1985) and synthesized by Merrifield solid-phase synthesis] with a peptide substrate [Val-Ser-Gln-Asn-Tyr-Pro-Ile-Val, 2 mg/mL when the reaction is initiated) were in 50 mM Na acetate, pH 5.5, at 30°C for 1 hr. Various concentrations of inhibitor in 1.0 µl DMSO were added to 36 µl of assay solution and the reaction initiated by the addition of 4 ul (1.5 µg) of synthetic protease. The reaction was quenched with 160 ul of 12% acetic acid. Products of the reaction were separated by HPLC (VYDAC wide pore 5 cm C-18 reverse phase, acetonitrile gradient, 0.1% trifluoroacetic acid). The extent of inhibition of the reaction was determined from the peak heights of the products. HPLC of the products, independently synthesized, provided quantitation standards and confirmation of the product composition. Results are shown in Table VII.

TABLE VII

| Compound | $IC_{50}$(nM) |
|---|---|
| A | 7 |
| B | 6 |
| C | 11 |
| D | 11 |
| E | 4 |

## EXAMPLE 8

Preparation of N-benzyl-N′-(succinoyl)-5(S)-amino-4(S)-hydroxy-6-phenyl-2(R)-(phenylmethyl)hexanoyl isoleucyl amide, Compound F

Step A: Preparation of N-benzyl-N′-(1,1-dimethylethoxycarbonyl)-5(S)-amino-4(S)-(1′,1′-dimethylethyl-1,1-dimethylsilyloxy)-6-phenyl-2(R)-(phenylmethyl)hexanoyl isoleucyl amide

The product of Example 1, Step H, 0.10 g (0.2 mmol) was dissolved in 2 ml of dry DMF, and to it was added 0.048 g (0.25 mmol) of EDC, 0.034 g (0.25 mmol) of HOBT and 0.077 g (0.3 mmol) of N-benzyl-isoleucyl amide hydrochloride. Triethylamine was added to the stirring solution until the pH was 8.5. After stirring for 18 hours, the reaction was poured into 20 ml of ice water and extracted with 3X20 ml of ethyl acetate. The combined organic extracts were washed with 1X20 ml of 10% citric acid, 1X20 ml of water, 1X20 ml of a saturated aqueous solution of $Na_2CO_3$, 20 ml of brine dried ($Na_2SO_4$), and the solvent removed to give 0.125 g (86%) of Step A product after purification by chromatography on silica gel ($CHCl_3$:$CH_3OH$, 99:1).

Step B: Preparation of N-benzyl-N′-(1,1-dimethylethoxycarbonyl)-5(S)-amino-4(S)-hydroxy-6-phenyl-2(R)-(phenylmethyl) hexanoyl isoleucyl amide

The Step A product, 0.115 g, was placed in a flask and to it was added 2 ml of a 1M solution of tetrabutylammonium fluoride in THF. After stirring for 4 hours, the solvent was removed in vacuo, and the residue was treated with 20 ml of ice water to precipitate the white solid product. Chromatography on silica gel ($CHCl_3$:$CH_3OH$, 99:1) provided 0.035 g (36%) of Step B product. mp = 204-206°C;

elemental analysis calc'd. for $C_{37}H_{49}N_3O_5$:

| | | | |
|---|---|---|---|
| | C, 72.17; | H, 8.02; | N, 6.82; |
| Found: | C, 72.47; | H, 8.30; | N, 6.73. |

Step C: Preparation of N-benzyl N′-(succinoyl)-5(S)-amino-4(S)-hydroxy-6-phenyl-2(R)-(phenylmethyl)hexanoyl isoleucyl amide

Removal of the Boc group and substituting thereto a succinoyl group was performed on the Step B product, 0.06 g (0.1 mmole), by the procedures of Example 9, both steps, to provide 0.03 g (49%) of the title compound. An analytical sample was obtained on recrystallization from acetonitrile;

elemental analysis calc'd for $C_{36}H_{45}N_3O_6$:

| | | | |
|---|---|---|---|
| | C, 70.22; | H, 7.37; | N, 6.82; |
| Found: | C, 70.20; | H, 7.40; | N, 6.77. |

## EXAMPLE 9

Preparation of N-(2(R)-hydroxy-1(S)-indanyl)-N′-(succinoyl)-5(S)-amino-4(S)-hydroxy-6-phenyl-2(R)-(phenylmethyl)-hexanoic carboxamide, Compound G

Step A: Preparation of N-(2(R)-hydroxy-1(S)-indanyl)-5(S)-amino 4(S)-hydroxy-6-phenyl-2(R)-(phenylmethyl)-hexanoic carboxamide

A 0.25 g (0.46 mmol) quantity of N-(2(R)-hydroxy-1(S)-indanyl)-N′-(1,1-dimethylethoxycarbonyl)-5(S)-amino-4(S)-hydroxy-6-phenyl-2(R)-(phenylmethyl)hexanoic carboxamide was dissolved in 20 ml of dry $CH_2Cl_2$ with stirring and cooling in an ice water bath under argon. To this solution was added 5 ml of trifluoroacetic acid, and the solution was stirred for 2 hours. The reaction mixture was concentrated in vacuo, and the residue partitioned between $CH_2Cl_2$ and saturated aqueous sodium bicarbonate solution. The organic layer was separated, washed with brine and dried over anhydrous $Na_2SO_4$, filtered and concentrated in vacuo to give 0.16 g (78%) of Step A product after chromatography on silica gel eluting with $CHCl_3$:$CH_3OH$, 95:5. An analytical sample was obtained by conversion to the maleate salt, mp 184-186°C;

elemental analysis, calcd. for $C_{28}H_{32}N_2O_3 \cdot C_4H_4O_4 \bullet H_2O$ (560.65):

| | | | |
|---|---|---|---|
| | C, 66.41; | H, 6.62; | N, 4.84; |
| Found: | C, 66.50; | H, 6.35; | N, 4.78; |

Step B: Preparation of N-(2(R)-hydroxy-1(S)-indanyl)-N′-(succinoyl)-5(S)-amino-4(S)-hydroxy-6-phenyl-2(R)-(phenylmethyl)-hexanoic carboxamide

A 0.050 g (0.089 mmol) quantity of N-(2(R)-hydroxy-1(S)-indanyl)-5(S)-amino-4(S)-hydroxy-6-phenyl-2(R)-(phenylmethyl)-hexanoic carboxamide was dissolved in 1 ml of dry DMF. To this solution was added 0.049 g (0.49 mmol) of succinic anhydride, and the reaction mixture was heated at 60° C for 8 hours. Ice water (20 ml) was added to the reaction mixture to precipitate 0.31 g (63%) of Step B product, mp 181-183°C after trituration with diethyl ether.

Elemental analysis, calc'd. for $C_{32}H_{36}N_2O_6$ (544.648):

| | | | |
|---|---|---|---|
| | C, 70.57; | H, 6.66; | N, 5.14; |
| Found: | C, 70.84; | H, 6.81; | N, 5.12. |

## EXAMPLE 10

Preparation of N-(2(R)-hydroxy-1(S)-indanyl)-N′-(methansulfonyl)-5(S)-amino-4(S)-hydroxy-6-phenyl-2(R)-(phenylmethyl)-hexanamide, Compound H.

Step A: Preparation of N-(2(R)-hydroxy-1(S)-indanyl)-5(S)-amino-4(S)-(1′,1′-dimethyl-ethyl-1,1-dimethylsilyoxy)-6-phenyl-2(R)-(phenylmethyl)-hexanamide

To a solution of 660 mgs. (0.99 mmol) of N-(2(R)-hydroxy-1(S)-indanyl)-5(S)-((1,1-dimethylethoxy)carbonyl)-amino)-4(S)-(1′,1′-dimethyl-ethyl-1,1-dimethylsilyoxy)-6-phenyl-2(R)-(phenylmethyl)-hexanamide dissolved in 30 mls of methylene chloride at 0° C was added 15 mls of trifluoroacetic acid. After 30 minutes, a thin layer chromatogram (TLC) showed that the reaction was complete. The reaction solution was concentrated, the residue dissolved in 100 mls of methylene chloride and transferred to a separatory funnel. The organic layer was washed with 2X50 ml of satd. $NaHCO_3$ solution and the organic layer was dried ($Na_2SO_4$), filtered and concentrated to give the crude Step A product which was pure enough to be used in the next reaction.

Step B: Preparation of N-(2(R)-hydroxy-1(S)-indanyl)-5(S)-(methanesulfonyl)amino-4(S)-1′,1′-dimethyl-ethyl-1,1-dimethylsilyoxy-6-phenyl-2(R)-(phenylmethyl)-hexanamide

To a solution of 55 mgs (0.10 mmol) of Step A product in 10 mls of methylene chloride was added 0.039 ml (0.276 mmol) of triethylamine and 0.032 mg (0.186 mmol) of methanesulfonic anhydride. The reaction was stirred at 23° C for 18 hours and worked up by diluting with 20 mls. of methylene chloride. The organic layer was washed with 2X20 mls of 10% citric acid soln., 20 mls of satd. $NaHCO_3$ solution, dried, filtered and concentrated to give the crude product. The residue was purified using prep. layer chromatography (10% MeOH/$CHCl_3$, $SiO_2$, 0.5 mm plate) to give 17 mgs of the Step B product compound as an oil.

Step C: Preparation of N-(2(R)-hydroxy-1(S)-indanyl)-5(S)-(methansulfonyl)amino-4(S)hydroxy-6-phenyl-2(R)-(phenylmethyl)-hexanamide

To a solution of 50 mgs. (0.078 mmol) of Step B product in 2 mls of THF was added 1 ml of a 1M solution of tetrabutylammonium fluoride (TBAF). After 1 hour an additional 0.5 ml of TBAF was added. After 2 hours, the reaction was diluted with water and the resultant precipitate was filtered and washed with water. The solid was dissolved in chloroform, dried ($Na_2SO_4$), filtered and concentrated. The crude product was chromatographed on a prep. plate (0.5 mm, 5% MeOH/$CHCl_3$) to give 24 mgs. of the Step C product, mp 180-182°C;

Anal. calc'd. for $C_{28}H_{34}N_2O_5S \bullet 0.25$ MeOH:

| | | | |
|---|---|---|---|
| | C, 66.20; | H, 6.65; | N, 5.28; |
| Found: | C, 65.96; | H, 6.59; | N, 5.65. |

## EXAMPLE 11

Preparation of
N-(2(R)-hydroxy-1(S)-indanyl)-N'-(5-oxo-2(S)-tetrahydrofuranylcarbonyl)-5(S)-amino-4(S)-hydroxy-6-phenyl-2(R)-(phenylmethyl)hexanamide, Compound J

(S)-(+)-5-Oxo-2-tetrahydrofurancarboxylic acid, 0.020 g, was dissolved in 1 mL of dry DMF, and to it was added 0.007 g of 1-hydroxybenztriazole hydrate, 0.010 g of 1-ethyl-3-(3-dimethylaminopropyl) carbodiimide hydrochloride and 0.020 g of N-(2(R)-hydroxy-1(S)-indanyl-5(S)-amino-4(S)-hydroxy-6-phenyl-2(R)-(phenylmethyl)hexanamide. Triethylamine was added to the stirring solution until the pH reached 8.5. After stirring for 12 hours at room temperature, the reaction was poured into 5 mL of water and extracted with 3X5 mL of ethyl acetate. The combined organic extracts were washed with 10% citric acid, water, saturated aqueous $NaHCO_3$ solution and dried over anhydrous $Na_2SO_4$. Evaporation of the solvent gave a residue which was chromatographed over silica gel (5% methanol/chloroform) to afford the title compound as a white solid.

## EXAMPLE 12

Assay for Inhibition of Microbial Expressed Viral Protease

Inhibition studies of the reaction of the protease expressed in Eschericia coli with a peptide substrate [Val-Ser-Gln-Asn-(betanapthyl)Ala-Pro-Ile-Val, 0.5 mg/ml at the time the reaction is initiated] were in 50 mM Na acetate, pH 5.5, at 30°C for 1 hr. Various concentrations of inhibitor in 1.0 ul DMSO were added to 25 ul of the peptide solution in water. The reaction is initiated by the addition of 15 ul of 0.33 nM protease (0.11 ng) in a solution of 0.133 M Na acetate pH 5.5 and 0.267% bovine serum albumin. The reaction was quenched with 160 ul of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently synthesized, proved quantitation standards and configurations of the product composition. Compounds F, G, H and J showed $IC_{50}$ values ranging from 0.5 to 10 nM.

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims and its equivalents thereof.

## Claims

1. Compounds of the formula:
A-G-B-B-J I,
wherein A is
1)
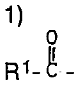 - wherein $R^1$ is
a) $C_{1-6}$ alkyl either unsubstituted or substituted with one or more of
i) $C_{1-4}$ alkyl;
ii) hydroxy;
iii) carboxy;
iv) halo wherein halo is F, Cl, Br, or I; except no halo on carbon adjacent to carbonyl;
v) amino;
vi) $C_{1-3}$ alkoxycarbonyl;
vii) $C_{1-3}$ alkoxy;
viii) $-CONR^2R^3$ wherein $R^2$ and $R^3$ are the same or different and are hydrogen, $C_{1-5}$ alkyl or $C_{1-5}$ alkoxyalkyl or joined together either directly to form a 5-7 membered heterocycle, or through a heteroatom selected from N, O, and S, to form a 6-membered heterocycle with the nitrogen to which they are attached;
ix) $-NR^2R^3$;
x) - 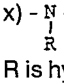 -P-$R^4$ wherein,
R is hydrogen or $C_{1-4}$ alkyl,
P is

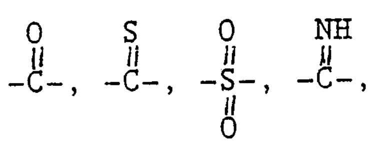

and

$R^4$ is H, $C_{1-3}$ alkyl, $C_{1-4}$ alkoxy, or $NR^2R^3$;

xi) $C_{3-7}$ cycloalkyl or $C_{6-10}$ aryl;

xii) 5 or 6 membered heterocycle, unsubstituted or substituted with OH, NH or $C_{1-4}$ alkyl; or

xiii) aryl of 6-10 carbon atoms, either unsubstituted or substituted with one or more of

(a) halo,

(b) hydroxy,

(c) $C_{1-3}$ alkoxy,

(d) $C_{1-3}$ alkyl,

(e) $-NR_2$, wherein R is defined above,

(f)

O

- C OR,

(g)

O

- C NR$_2$,

(h) $-SO_2NR_2$,

(i) $-CH_2NR_2$,

(j)

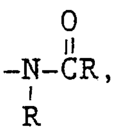

or

(k) - N SO$_2$R;

R

xiv) $-OSiR^3(R^2)_2$

b) aryl of 6-10 carbon atoms, either unsubstituted or substituted with one or more of

i) $C_{1-4}$ alkyl,

ii) $C_{1-3}$ alkoxy,

iii) hydroxy, or

iv) halo;

v) $-NR_2$,

vi)

O

- C OR,

vii)

O

- C NR$_2$,

viii) $-SO_2NR_2$,

ix) $-CH_2NR_2$,

x)

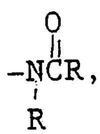

or

xi) - N SO$_2$R;

R

c) 5 or 6 membered heterocycle;

2) $R^1-SO_2-$, except $R^1$ is nor aryl,

3)

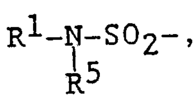

wherein

$R^5$ is H or $C_{1-5}$ alkyl or joined together with $R^1$ either directly to form 5-7 membered heterocycle, or through a heteroatom selected from N, O, and S, to form a 6-membered heterocycle with the nitrogen to which they are attached;

4)

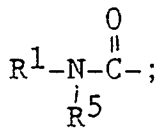

5)

$R^1-S-\overset{O}{\overset{\|}{C}}-$;

6) $(R^1-)_q$ wherein q is 1 or 2;

G is

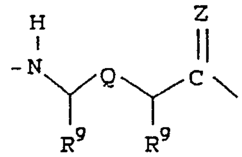

or

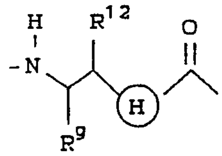

,

wherein Z is O, S, or HH, and

$R^9$ is independently

1) hydrogen;

2)

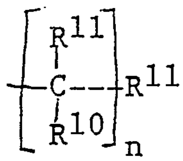

3) -OR, wherein R is H, or $C_{1-4}$ alkyl

4) $-NR_2$,

5) $-C_{1-4}$ alkylene-$R^{11}$;

wherein n is 0-5 and $R^{10}$ is independently

a) hydrogen,

b) hydroxy, or

c) $C_{1-4}$-alkyl;

$R^{11}$ is

a) hydrogen,

b) aryl, unsubstituted or substituted with one or more of

i) halo,

ii) hydroxy,

iii) $-NH_2$, $-NO_2$, -NHR, or $-NR_2$, wherein R is H, or $C_{1-4}$ alkyl,

iv) $C_{1-4}$ alkyl,

v) $C_{1-3}$ alkoxy,

vi) -COOR,

vii) $-\overset{O}{\overset{\|}{C}}NR_2$,

viii) $-CH_2NR_2$,

ix) $-CH_2NH\overset{O}{\overset{\|}{C}}R$,

x) CN,

xi) $CF_3$,

xii) $-NH\overset{O}{\overset{\|}{C}}R$,

xiii) aryl $C_{1-3}$ alkoxy,

xiv) aryl,

xv) $-NRSO_2R$,

xvi) $-OP(O)(OR_x)_2$ wherein $R_x$ is H or aryl, or

xvii) $-O-\overset{O}{\overset{\|}{C}}-C_{1-4}$ alkyl substituted with one or more of amine or quaternary amine;

c) 5 or 6 membered heterocycle including up to 3 heteroatoms selected from N, O, and S, any of which heterocycle may be unsubstituted or substituted with one or more of
i) halo,
ii) hydroxy,
iii) $-NH_2$, $-NHR$, $-NR_2$,
iv) $C_{1-4}$ alkyl,
v) $C_{1-3}$ alkoxy,
vi) -COOR,
vii)
$-\overset{O}{\overset{\parallel}{C}}NR_2$,
viii) $-CH_2NR_2$,
ix) $-NH\underset{O}{\underset{\parallel}{C}}R$,
x) -CN,
xi) $CF_3$,
xii) $-NHSO_2R$,
xiii) $-OP(O)(OR_x)_2$ wherein $R_x$ is H or aryl, or
xiv)
$-O-\overset{O}{\overset{\parallel}{C}}-C_{1-4}$ alkyl substituted with one or more of amine or quaternary amine;
d) $C_{1-6}$ alkyl or $C_{1-6}$ alkenyl, unsubstituted or substituted with one or more of
i) hydroxy,
ii) $C_{1-4}$ alkyl,
iii) $-NH_2$, $-NHR$, $-NR_2$,
iv)
$-NH\overset{NH}{\overset{\parallel}{CH}}$ ,
v)
$-NH-\overset{NH}{\overset{\parallel}{C}}-NH_2$,
vi) -COOH,
vii)
$-\overset{O}{\overset{\parallel}{C}}OR$,
viii) -SR or arylthio,
ix) $-SO_2NHR$,
x) $C_{1-4}$ alkyl sulfonyl amino or aryl sulfonyl amino,
xi) -CONHR,
xii)
$-NH\overset{O}{\overset{\parallel}{C}}R$,
xiii) -OR
xiv) aryl $C_{1-3}$ alkoxy, or,
xv) aryl;
e) $C_{3-7}$ cycloalkyl unsubstituted or substituted with one or more of
i) hydroxy,
ii) $C_{1-4}$alkyl,
iii) $-NH_2$, $-NHR$, $-NHR_2$,
iv)
$-NH-\overset{NH}{\overset{\parallel}{CH}}$ ,
v)
$-NH-\overset{NH}{\overset{\parallel}{C}}-NH_2$,
vi) -COOH,
vii)
$-\overset{O}{\overset{\parallel}{C}}-OR$,
viii) -SR,
ix) $-SO_2NH_2$,
x) alkyl sulfonylamino or aryl sulfonylamino,
xi) -CONHR, or
xii)

$-NH\overset{O}{\overset{\|}{C}}R$;

f) a 5- to 7-membered carbocyclic or 7- to 10-membered bicyclic carbocyclic ring which is either saturated or unsaturated, said carbocyclic ring being unsubstituted or substituted with one or more of

i) halo

ii) -OR, wherein R is H or $C_{1-4}$ alkyl,

iii) $-\overset{O}{\overset{\|}{C}}OR$,

iv) $-\overset{O}{\overset{\|}{C}}NR_2$,

v) $-CH_2NR_2$,

vi) $-SO_2NR_2$ or $-S(O)_y R$ wherein y is 0, 1, or 2;

vii) $-NR_2$,

viii) $-NH\overset{O}{\overset{\|}{C}}R$,

ix) $C_{1-4}$ alkyl,

x) phenyl,

xi) $-CF_3$, or

xii) $-\overset{R}{\overset{|}{N}}-SO_2R$;

g) benzofuryl; indolyl; azabicyclo $C_{7-11}$ cycloalkyl; or benzopiperidinyl;

$R^{12}$ is -OH or $-NHR^{13}$, wherein $R^{13}$ is -H, $-\overset{O}{\overset{\|}{C}}H$, $-C_{1-4}$-alkyl or -COOR; and

Ⓗ is

1) $C_{3-7}$ cycloalkyl either unsubstituted or substituted with one or more of

a) $C_{1-4}$alkyl,

b) hydroxy,

c) $-NR_2$,

d) -COOR,

e) CONHR,

f) $-NHSO_2R$,

g) $-NH\overset{O}{\overset{\|}{C}}R$,

h) aryl,

i) aryl substituted with $C_{1-4}$ alkyl,

j) heterocycle, or

k) heterocycle substituted with $C_{1-4}$ alkyl;

2) phenyl, either unsubstituted or substituted with one or more of

a) hydroxy,

b) -OR,

c) $-NHR^{13}$,

d) -COOR,

e) $-\overset{O}{\overset{\|}{C}}NR_2$, or

f) $-NH\overset{O}{\overset{\|}{C}}R$; or

3) 5 to 7-membered heterocycle, any of which heterocycle may be unsubstituted or substituted with one or more of

i) halo,

ii) hydroxy,

iii) $NR_2$, or

iv) $C_{1-4}$ alkyl;

Q is

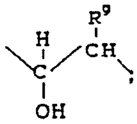 ; 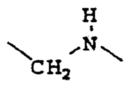 ; 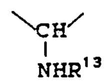 ; 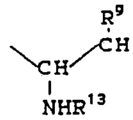 ; 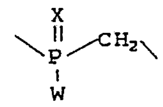 , 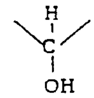 ,

wherein $R^9$ and $R^{13}$ are defined above; X is O, S, or NH; and
W is
1) OH,
2) $NH_2$,
3) OR, or
4) NHR;
B is, independently, absent, or

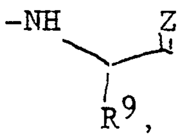 ,

J is
1) $YR^{14}$ wherein:
Y is O or NH, and
$R^{14}$ is
a) H;
b) $C_{1-6}$ alkyl, unsubstituted or substituted with one or more of
i) $-NR^2_2$,
ii) -OR,
iii) $-NHSO_2C_{1-4}$ alkyl,
iv) $-NHSO_2$ aryl, or $-NHSO_2$ (dialkylaminoaryl),
v) $-CH_2OR$,
vi) $-C_{1-4}$ alkyl,
vii)
$-\overset{O}{\overset{\|}{C}}OR$,
viii) $-\overset{O}{\overset{\|}{C}}NR_2$,
ix)

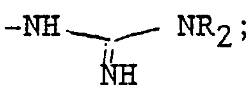 ; 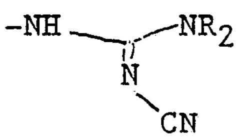 ,

x)
$-NH\overset{O}{\overset{\|}{C}}R$,
xi)

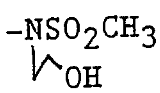 ,

xii)

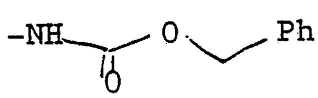 ,

xiii) $-NR_3^{\oplus}A^{\ominus}$ wherein $A^{\ominus}$ is a counterion,
xiv) $-NR^{15}R^{16}$ wherein $R^{15}$ and $R^{16}$ are the same or different and are $C_{1-5}$ alkyl joined together directly to form a 5-7 membered heterocycle,
xv) aryl,
xvi) -CHO,
xvii) $-OP(O)(OR_x)_2$ wherein $R_x$ is H or aryl, or
xviii)
$-O-\overset{O}{\overset{\|}{C}}-C_{1-4}$ alkyl substituted with one or more of amine or quaternary amine;

c) $-(CH_2CH_2O)CH_3$ or $-(CH_2CH_2O)_nH$;
2) $-N(R^{14})_2$;
3) $-NR^{15}R^{16}$ wherein $R^{15}$ and $R^{16}$ are defined above;
4)

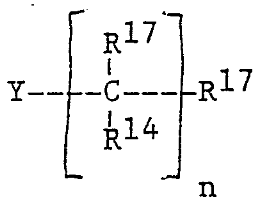

wherein:
Y, $R^{14}$, and n are defined above, and
$R^{17}$ is
a) hydrogen;
b) aryl unsubstituted or substituted with one or more of
i) halo,
ii) -OR, wherein R is H or $C_{1-4}$ alkyl,
iii) $-\overset{O}{\overset{\|}{C}}OR$,
iv) $-\overset{O}{\overset{\|}{C}}NR_2$,
v) $-CH_2NR_2$,
vi) $-SO_2NR_2$,
vii) $-NR^2_2$,
viii) $-NH\overset{O}{\overset{\|}{C}}R$,
ix) $C_{1-4}$ alkyl,
x) phenyl,
xi) $-CF_3$,
xii) $-\overset{R}{\overset{|}{N}}-SO_2R$,
xiii) $-C_{1-4}$ alkyl-$NR_2$,
xiv) $-OP(O)(OR_x)_2$ wherein $R_x$ is H or aryl, or
xv) $-O-\overset{O}{\overset{\|}{C}}-C_{1-4}$ alkyl substituted with one or more of amine or quaternary amine;
c) heterocycle, unsubstituted or substituted with one or more of
i) halo,
ii) -OR, wherein R is H, $C_{1-4}$ alkyl, or $C_{1-4}$ alkenyl,
iii) $-\overset{O}{\overset{\|}{C}}OR$,
iv) $-\overset{O}{\overset{\|}{C}}NR_2$,
v) $-CH_2NR_2$,
vi) $-SO_2NR_2$,
vii) $-NR_2$,
viii) $-NH\overset{O}{\overset{\|}{C}}R$,
ix) $C_{1-4}$ alkyl,
x) phenyl,
xi) $-CF_3$,
xii) $-\overset{R}{\overset{|}{N}}-SO_2R$,
xiii) phenyl $C_{1-4}$ alkyl,

xiv) $-O\overset{O}{\overset{\|}{C}}R$,

xv) $-OP(O)(OR_x)_2$ wherein $R_x$ is H or aryl, or

xvi) $-O-\overset{O}{\overset{\|}{C}}-C_{1-4}$ alkyl substituted with one or more amine or quaternary amine;

d) A 5- to 7-membered carbocyclic or 7- to 10-membered bicyclic carbocyclic ring which is either saturated or unsaturated, the carbocyclic ring being unsubstituted or substituted with one or more of

i) halo,

ii) -OR, wherein R is H or $C_{1-4}$ alkyl,

iii) $-\overset{O}{\overset{\|}{C}}OR$,

iv) $-\overset{O}{\overset{\|}{C}}NR_2$,

v) $-CH_2NR_2$,

vi) $-SO_2NR_2$,

vii) $-NR_2$,

viii) $-NH\overset{O}{\overset{\|}{C}}R$,

ix) $C_{1-4}$ alkyl,

x) phenyl,

xi) $-CF_3$,

xii) $-\overset{R}{\overset{|}{N}}-SO_2R$,

xiii) $-OP(O)(OR_x)_2$ wherein $R_x$ is H or aryl, or

xiv) $-O-\overset{O}{\overset{\|}{C}}-C_{1-4}$ alkyl substituted with one or more of amine or quaternary amine;

or pharmaceutically acceptable salts thereof.

2. The compounds of Claim 1 wherein B is independently present twice and Z is O.

3. The compounds of Claim 2 wherein J is $NH_2$, and Q is

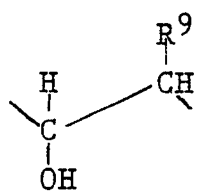

.

4. The compounds of Claim 2 wherein J is $NH_2$, and Q is

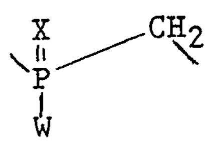

.

5. The compounds of Claim 2 wherein J is $NH_2$, and Q is

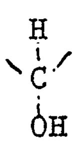

.

6. The compounds of Claim 1 wherein B is present once and Z is O.

7. The compounds of Claim 6 wherein Q is

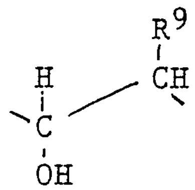 .

8. The compounds of Claim 6 wherein Q is

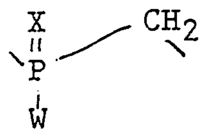 .

9. The compounds of Claim 6 wherein Q is

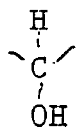 .

10. The compounds of Claim 1 wherein B is always absent.
11. The compounds of Claim 10 wherein Q is

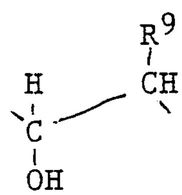 .

12. The compounds of Claim 10 wherein Q is

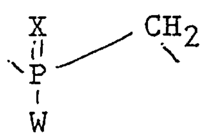 .

13. The compounds of Claim 10 wherein Q is

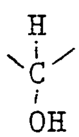 .

14. The compounds of Claim 1 wherein G is:

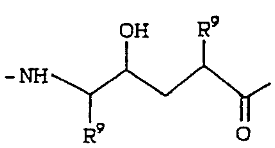 or 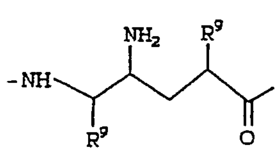 .

15. Compounds of Claim 1 wherein G is:

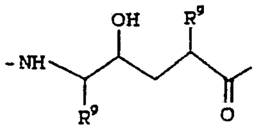 or 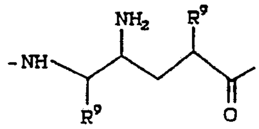 ;

and B is absent or present once.

16. Compounds of Claim 1 wherein G is

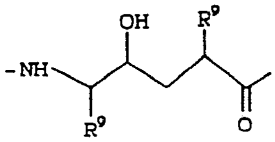 or 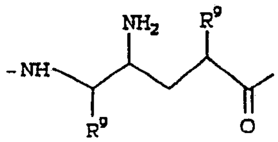 ;

B is absent or present once; and

J is 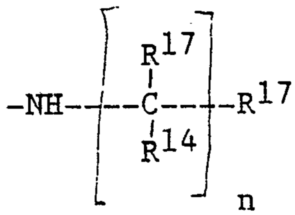 .

17. Compounds of Claim 1 wherein:

A is

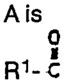 or $R^1$-$SO_2$-, with the proviso that $R^1$ is not aryl when attached to S;

G is:

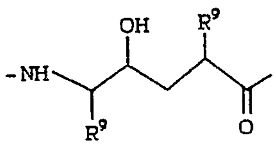 or 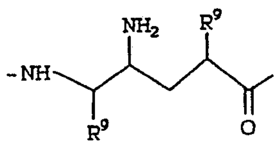 ;

B is absent or present once; and

J is 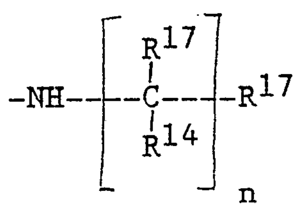 .

18. N′-(1,1-dimethylethylaminocarbonyl)-5(S)-amino-4(S)-hydroxy-6-phenyl-2(R)-(phenylmethyl)hexanoyl-leucyl-phenylalanylamide, or pharmaceutically acceptable salt thereof.

19 N′-(2,2-dimethylpropanoyl)-5(S)-amino-4(S)-hydroxy-6-phenyl-2(R)-(phenylmethyl)hexanoyl-leucyl-phenylalanylamide, or pharmaceutically acceptable salt thereof.

20. N′-(2-thienoyl)-5(S)amino-4(S)-hydroxy-6-phenyl-2(R)-(phenylmethyl)hexanoyl-leucyl-phenylalanylamide, or pharmaceutically acceptable salt thereof.

21. N′-(3,3-dimethylbutanoyl)-5(S)-amino-4(S)-hydroxy-6-phenyl-2(R)-(phenylmethyl)hexanoyl-leucyl-phenylalanylamide, or pharmaceutically acceptable salt thereof.

22. N′-(3-phenylpropanoyl)-5(S)-amino-4(S)-hydroxy-6-phenyl-2(R)-(phenylmethyl)hexanoyl-leucyl-phenylalanylamide, or pharmaceutically acceptable salt thereof.

23. N-benzyl-N′-(succinoyl)-5(S)-amino-4(S)-hydroxy-6-phenyl-2(R)-(phenylmethyl)hexanoyl isoleucyl amide;

N-(2(R)-hydroxy-1(S)-indanyl)-N′-(succinoyl)-5(S)-amino-4(S)-hydroxy-6-phenyl-2(R)-(phenylmethyl)hexanamide;

N-(2(R)-hydroxy-1(S)-indanyl)-N′-(methanesulfonyl)-5(S)-amino-4(S)-hydroxy-6-phenyl-2(R)-(phenylmethyl)hexanamide;

N-(2(R)-hydroxy-1(S)-indanyl)-N′-(5-oxo-2(S)-tetrahydrofurancarbonyl)-5(S)-amino-4(S)-hydroxy-6-phenyl-2(R)-(phenylmethyl)hexanamide,

N'-(2,2-dimethylpropanoyl)-5(S)-amino-4(S)-hydroxy-6-phenyl-2(R)-(phenylmethyl) hexanoyl-Leu-Phe-amide,
N'-(1,1-dimethylethylaminocarbonyl)-5(S)-amino-4(S)-hydroxy-2(R)-phenylmethyl-hexanoyl -Leucyl-Phenylalanyl-amide,
N'-(3-phenylpropanoyl)-5(S)-amino-4(S)-hydroxy-6-phenyl-2(R)-(Phenylmethyl)-hexanoyl-Leucyl-Phenylalanyl-amide,
N'-(Succinoyl)-5(S)-amino-4(S)-hydroxy-6-phenyl-2(R)-(phenylmethyl)hexanoyl-N-(phenylmethyl)-Ile-amide,
N-(2(R)-hydroxy-1(S)-indanyl)-N'-(4-t-butyldimethylsilyloxybutanoyl)-5(S)-amino-4(S)-hydroxy-6-phenyl-2(R)-(phenylmethyl)hexanamide,
N-(2(R)-hydroxy-1(S)-indanyl)-N'-(methanesulfonyl)-5(S)-amino-4(S)-hydroxy-6-phenyl-2(R)-(phenylmethyl)hexanamide,
N-(2(R)-hydroxy-1(S)-indanyl)-N'-(4-hydroxybutanoyl)-5(S)-amino-4(S)-hydroxy-6-phenyl-2(R)-phenylmethyl hexanamide,
N'-(methanesulfonyl)-5(S)-amino-4(S)-hydroxy-6-phenyl-2(R)-(phenylmethyl)hexanoyl-N-[2,3-dihydroxypropyl]-Val-amide, or
N-(2(R)-hydroxy-1(S)-indanyl)-N'-[2-(2-[2-methoxyethoxy]ethoxy)ethoxycarbonyl]-5(S)-amino-2(R)-benzyl-4(S)-hydroxy-6-phenyl hexanamide,
or pharmaceutically acceptable salts thereof.

24. The compounds of Claims 1-23, in combination with any of the antivirals, immunomodulators, antibiotics or vaccines of Table VI.

25. A pharmaceutical composition comprising the compounds of Claims 1-23, and a pharmaceutically acceptable carrier.

26. A pharmaceutical composition comprising the compound in combination according to Claim 24, and a pharmaceutically acceptable carrier.

27. The pharmaceutical composition of Claim 25, for use in the treatment of AIDS, in the prevention of infection by HIV, in the treatment of infection by HIV, or in the inhibition of HIV protease.

28. The pharmaceutical composition of Claim 26, for use in the treatment of AIDS, in the prevention of infection by HIV, in the treatment of infection by HIV, or in the inhibition of HIV protease.

29. The use of a compound of any one of Claims 1-23, for the preparation of a medicament useful for treating AIDS.

30. The use of a combination according to Claim 24, for the preparation of a medicament useful for treating AIDS.

31. The use of a compound as claimed in any one of Claims 1-23, for the preparation of a medicament useful for preventing infection by HIV.

32. The use of the combination as claimed in Claim 24 for the preparation of a medicament useful for preventing infection by HIV.

33. The use of a compound as claimed in any one of claims 1-23 for the preparation of a medicament useful for treating infection by HIV.

34. The use of the combination as claimed in Claim 24 for the preparation of a medicament useful for treating infection by HIV.

35. The use of a compound as claimed in any one of claims 1-23 for the preparation of a medicament useful for inhibiting HIV protease.

36. The use of the combination as claimed in Claim 24 for the preparation of a medicament useful for inhibiting HIV protease.

**Claims for the following Contracting States: ES, GR**

1.- A process for preparing HIV protease inhibitors, useful for the treatment of AIDS, having formula I
A-G-B-B-J I,
wherein A is
1)
$R^1-\overset{O}{\overset{\|}{C}}-$ wherein $R^1$ is
a) $C_{1-6}$ alkyl either unsubstituted or substituted with one or more of
i) $C_{1-4}$ alkyl;
ii) hydroxy;
iii) carboxy;
iv) halo wherein halo is F, Cl, Br, or I; except no halo on carbon adjacent to carbonyl;
v) amino;
vi) $C_{1-3}$ alkoxycarbonyl;
vii) $C_{1-3}$ alkoxy;
viii) $-CONR^2R^3$ wherein $R^2$ and $R^3$ are the same or different and are hydrogen, $C_{1-5}$ alkyl or $C_{1-5}$ alkoxyalkyl or joined together either directly to form a 5-7 membered heterocycle, or through a heteroatom selected from N, O, and S, to form a 6-membered heterocycle with the nitrogen to which they

are attached;
ix) $-NR^2R^3$;
x) $-\underset{R}{N}-P-R^4$ wherein,
R is hydrogen or $C_{1-4}$ alkyl,
P is

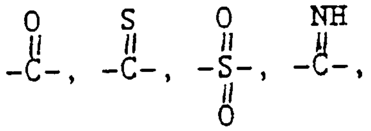

and
$R^4$ is H, $C_{1-3}$ alkyl, $C_{1-4}$ alkoxy, or $NR^2R^3$;
xi) $C_{3-7}$ cycloalkyl or $C_{6-10}$ aryl;
xii) 5 or 6 membered heterocycle, unsubstituted or substituted with OH, NH or $C_{1-4}$ alkyl; or
xiii) aryl of 6-10 carbon atoms, either unsubstituted or substituted with one or more of
(a) halo,
(b) hydroxy,
(c) $C_{1-3}$ alkoxy,
(d) $C_{1-3}$ alkyl,
(e) $-NR_2$, wherein R is defined above,
(f)
$-\overset{O}{C}OR$,
(g)
$-\overset{O}{C}NR_2$,
(h) $-SO_2NR_2$,
(i) $-CH_2NR_2$,
(j)

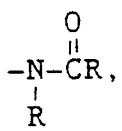

or
(k) $-\underset{R}{N}SO_2R$;
xiv) $-OSiR^3(R^2)_2$
b) aryl of 6-10 carbon atoms, either unsubstituted or substituted with one or more of
i) $C_{1-4}$ alkyl,
ii) $C_{1-3}$ alkoxy,
iii) hydroxy, or
iv) halo;
v) $-NR_2$,
vi)
$-\overset{O}{C}OR$,
vii)
$-\overset{O}{C}NR_2$,
viii) $-SO_2NR_2$,
ix) $-CH_2NR_2$,
x)

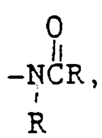

or
xi) $-\underset{R}{N}SO_2R$;

c) 5 or 6 membered heterocycle;
2) $R^1$-$SO_2$-, except $R^1$ is nor aryl,
3)

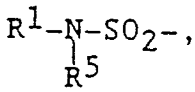

wherein
$R^5$ is H or $C_{1-5}$ alkyl or joined together with $R^1$ either directly to form 5-7 membered heterocycle, or through a heteroatom selected from N, O, and S, to form a 6-membered heterocycle with the nitrogen to which they are attached;
4)

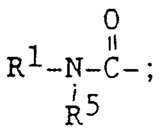

5)

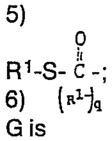

6) $(R^1\text{-})_q$ wherein q is 1 or 2;
G is

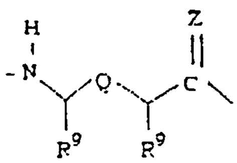

or

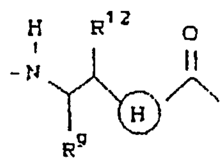

.

wherein Z is O, S, or HH, and
$R^9$ is independently
1) hydrogen;
2)

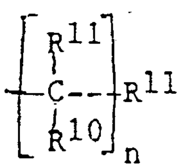

3) -OR, wherein R is H, or $C_{1-4}$ alkyl
4) -$NR_2$,
5) -$C_{1-4}$ alkylene-$R^{11}$;
wherein n is 0-5 and $R^{10}$ is independently
a) hydrogen,
b) hydroxy, or
c) $C_{1-4}$-alkyl;
$R^{11}$ is
a) hydrogen,
b) aryl, unsubstituted or substituted with one or more of
i) halo,
ii) hydroxy,
iii) -$NH_2$, -$NO_2$, -NHR, or -$NR^2$, wherein R is H, or $C_{1-4}$ alkyl,
iv) $C_{1-4}$ alkyl,
v) $C_{1-3}$ alkoxy,
vi) -COOR,
vii)

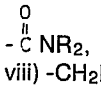

viii) -$CH_2NR_2$,
ix)

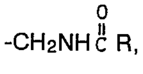

x) CN,
xi) $CF_3$,
xii)
$-NH\overset{O}{\overset{\|}{C}}R$,
xiii) aryl $C_{1-3}$ alkoxy,
xiv) aryl,
xv) $-NRSO_2R$,
xvi) $-OP(O)(OR_x)_2$ wherein $R_x$ is H or aryl, or
xvii)
$-O-\overset{O}{\overset{\|}{C}}-C_{1-4}$ alkyl substituted with one or more of amine or quaternary amine;
c) 5 or 6 membered heterocycle including up to 3 heteroatoms selected from N, O, and S, any of which heterocycle may be unsubstituted or substituted with one or more of
i) halo,
ii) hydroxy,
iii) $-NH_2$, -NHR, $-NR_2$,
iv) $C_{1-4}$ alkyl,
v) $C_{1-3}$ alkoxy,
vi) -COOR,
vii)
$-\overset{O}{\overset{\|}{C}}NR_2$,
viii) $-CH_2NR_2$,
ix) $-NH\underset{O}{\underset{\|}{C}}R$,
x) -CN,
xi) $CF_3$,
xii) $-NHSO_2R$,
xiii) $-OP(O)(OR_x)_2$ wherein $R_x$ is H or aryl, or
xiv)
$-O-\overset{O}{\overset{\|}{C}}-C_{1-4}$ alkyl substituted with one or more of amine or quaternary amine;
d) $C_{1-6}$ alkyl or $C_{1-6}$ alkenyl, unsubstituted or substituted with one or more of
i) hydroxy,
ii) $C_{1-4}$ alkyl,
iii) $-NH_2$, -NHR, $-NR_2$,
iv)
$-NH\overset{NH}{\overset{\|}{CH}}$ ,
v)
$-NH-\overset{NH}{\overset{\|}{C}}-NH_2$,
vi) -COOH,
vii)
$-\overset{O}{\overset{\|}{C}}OR$,
viii) -SR or arylthio,
ix) $-SO_2NHR$,
x) $C_{1-4}$ alkyl sulfonyl amino or aryl sulfonyl amino,
xi) -CONHR,
xii)
$-NH\overset{O}{\overset{\|}{C}}R$,
xiii) -OR
xiv) aryl $C_{1-3}$ alkoxy, or,
xv) aryl;
e) $C_{3-7}$ cycloalkyl unsubstituted or substituted with one or more of
i) hydroxy,
ii) $C_{1-4}$alkyl,
iii) $-NH_2$, -NHR, $-NHR_2$,
iv)
$-NH-\overset{NH}{\overset{\|}{CH}}$ ,
v)

$-NH-\overset{\overset{NH}{\parallel}}{C}-NH_2$,
vi) -COOH,
vii)
$-\overset{\overset{O}{\parallel}}{C}-OR$,
viii) -SR,
ix) $-SO_2NH_2$,
x) alkyl sulfonylamino or aryl sulfonylamino,
xi) -CONHR, or
xii)
$-NH\overset{\overset{O}{\parallel}}{C}R$;
f) a 5- to 7-membered carbocyclic or 7- to 10-membered bicyclic carbocyclic ring which is either saturated or unsaturated, said carbocyclic ring being unsubstituted or substituted with one or more of
i) halo
ii) -OR, wherein R is H or $C_{1-4}$ alkyl,
iii)
$-\overset{\overset{O}{\parallel}}{C}OR$,
iv)
$-\overset{\overset{O}{\parallel}}{C}NR_2$,
v) $-CH_2NR_2$,
vi) $-SO_2NR_2$ or $-S(O)_yR$ wherein y is 0, 1, or 2;
vii) $-NR_2$,
viii)
$-NH\overset{\overset{O}{\parallel}}{C}R$,
ix) $C_{1-4}$ alkyl,
x) phenyl,
xi) $-CF_3$, or
xii)
$-\overset{\overset{R}{|}}{N}-SO_2R$;
g) benzofuryl; indolyl; azabicyclo $C_{7-11}$ cycloalkyl; or benzopiperidinyl;
$R^{12}$ is -OH or $-NHR^{13}$, wherein $R^{13}$ is -H,
$-\overset{\overset{O}{\parallel}}{C}H$, $-C_{1-4}$-alkyl or -COOR; and
Ⓗ is
1) $C_{3-7}$ cycloalkyl either unsubstituted or substituted with one or more of
a) $C_{1-4}$alkyl,
b) hydroxy,
c) $-NR_2$,
d) -COOR,
e) CONHR,
f) $-NHSO_2R$,
g)
$-NH\overset{\overset{O}{\parallel}}{C}R$,
h) aryl,
i) aryl substituted with $C_{1-4}$ alkyl,
j) heterocycle, or
k) heterocycle substituted with $C_{1-4}$ alkyl;
2) phenyl, either unsubstituted or substituted with one or more of
a) hydroxy,
b) -OR,
c) $-NHR^{13}$,
d) -COOR,
e)
$-\overset{\overset{O}{\parallel}}{C}NR_2$, or
f)
$-NH\overset{\overset{O}{\parallel}}{C}R$; or

3) 5 to 7-membered heterocycle, any of which heterocycle may be unsubstituted or substituted with one or more of
i) halo,
ii) hydroxy,
iii) $NR_2$, or
iv) $C_{1-4}$ alkyl;
Q is

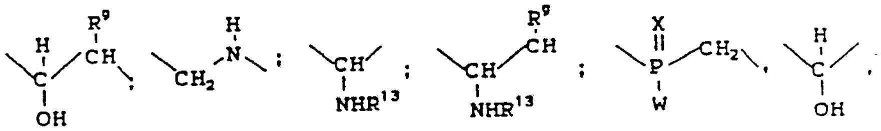

wherein $R^9$ and $R^{13}$ are defined above; X is O, S, or NH; and
W is
1) OH,
2) $NH_2$,
3) OR, or
4) NHR;
B is, independently, absent, or

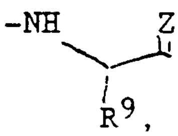

J is
1) $YR^{14}$ wherein:
Y is O or NH, and
$R^{14}$ is
a) H;
b) $C_{1-6}$ alkyl, unsubstituted or substituted with one or more of
i) $-NR^2_2$,
ii) -OR,
iii) $-NHSO_2C_{1-4}$ alkyl,
iv) $-NHSO_2$ aryl, or $-NHSO_2$ (dialkylaminoaryl),
v) $-CH_2OR$,
vi) $-C_{1-4}$ alkyl,
vii)
$-\overset{O}{\overset{\parallel}{C}}OR$,
viii)
$-\overset{O}{\overset{\parallel}{C}}NR_2$,
ix)

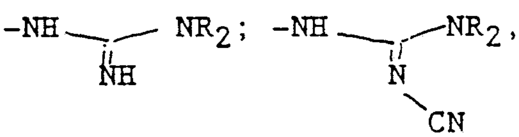

x)
$-NH\overset{O}{\overset{\parallel}{C}}R$,
xi)

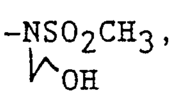

xii)

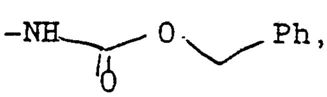

xiii) $-NR_3^{\oplus}A^{\ominus}$ wherein $A^{\ominus}$ is a counterion,
xiv) $-NR^{15}R^{16}$ wherein $R^{15}$ and $R^{16}$ are the same or different and are $C_{1-5}$ alkyl joined together directly to form a 5-7 membered heterocycle,
xv) aryl,
xvi) -CHO,
xvii) $-OP(O)(OR_x)_2$ wherein $R_x$ is H or aryl, or
xviii)
$-O-\overset{\underset{\|}{O}}{C}-C_{1-4}$ alkyl substituted with one or more of amine or quaternary amine;
c) $-(CH_2CH_2O)CH_3$ or $-(CH_2CH_2O)_nH$;
2) $-N(R^{14})_2$;
3) $-NR^{15}R^{16}$ wherein $R^{15}$ and $R^{16}$ are defined above;
4)

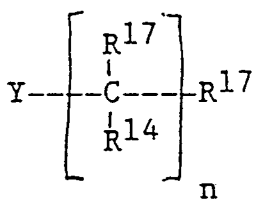

wherein:
Y, $R^{14}$, and n are defined above, and
$R^{17}$ is
a) hydrogen;
b) aryl unsubstituted or substituted with one or more of
i) halo,
ii) -OR, wherein R is H or $C_{1-4}$ alkyl,
iii)
$-\overset{\underset{\|}{O}}{C}OR$,
iv)
$-\overset{\underset{\|}{O}}{C}NR_2$,
v) $-CH_2NR_2$,
vi) $-SO_2NR_2$,
vii) $-NR^2_2$,
viii)
$-NH\overset{\underset{\|}{O}}{C}R$,
ix) $C_{1-4}$ alkyl,
x) phenyl,
xi) $-CF_3$,
xii)
$-\overset{R}{\overset{|}{N}}-SO_2R$,
xiii) $-C_{1-4}$ alkyl-$NR_2$,
xiv) $-OP(O)(OR_x)_2$ wherein $R_x$ is H or aryl, or
xv)
$-O-\overset{\underset{\|}{O}}{C}-C_{1-4}$ alkyl substituted with one or more of amine or quaternary amine;
c) heterocycle, unsubstituted or substituted with one or more of
i) halo,
ii) -OR, wherein R is H, $C_{1-4}$ alkyl, or $C_{1-4}$ alkenyl,
iii)
$-\overset{\underset{\|}{O}}{C}OR$,
iv)
$-\overset{\underset{\|}{O}}{C}NR_2$,
v) $-CH_2NR_2$,
vi) $-SO_2NR_2$,
vii) $-NR_2$,
viii)

$-NH\overset{O}{\overset{\|}{C}}R$,
ix) $C_{1-4}$ alkyl,
x) phenyl,
xi) $-CF_3$,
xii)
$-\overset{R}{\overset{|}{N}}-SO_2R$,
xiii) phenyl $C_{1-4}$ alkyl,
xiv)
$-O\overset{O}{\overset{\|}{C}}R$,
xv) $-OP(O)(OR_x)_2$ wherein $R_x$ is H or aryl, or
xvi)
$-O-\overset{O}{\overset{\|}{C}}-C_{1-4}$ alkyl substituted with one or more amine or quaternary amine;
d) A 5- to 7-membered carbocyclic or 7- to 10-membered bicyclic carbocyclic ring which is either saturated or unsaturated, the carbocyclic ring being unsubstituted or substituted with one or more of
i) halo,
ii) -OR, wherein R is H or $C_{1-4}$ alkyl,
iii)
$-\overset{O}{\overset{\|}{C}}OR$,
iv)
$-\overset{O}{\overset{\|}{C}}NR_2$,
v) $-CH_2NR_2$,
vi) $-SO_2NR_2$,
vii) $-NR_2$,
viii)
$-NH\overset{O}{\overset{\|}{C}}R$,
ix) $C_{1-4}$ alkyl,
x) phenyl,
xi) $-CF_3$,
xii)
$-\overset{R}{\overset{|}{N}}-SO_2R$,
xiii) $-OP(O)(OR_x)_2$ wherein $R_x$ is H or aryl, or
xiv)
$-O-\overset{O}{\overset{\|}{C}}-C_{1-4}$ alkyl substituted with one or more of amine or quaternary amine;
or pharmaceutically acceptable salts thereof,
said process being characterized by
a) forming the desired G component duly protected on its amine group;
b) coupling on to the carboxy terminal end of said protected G component the other components B (if present) and/or J, either one by one or together, each component being duly protected when necessary by protecting groups which are adequate to both the particular coupling conditions and the aminoacid components and analogs thereof involved in the synthesis, by forming amide bonds by any coupling method of either solution-phase or solid-phase peptide synthesis;
c) removing the amine protecting group originally contained in the G component and adding the amine protecting group not useful or commonly employed in peptide synthesis (A) by reaction of the amine obtained with the desired A-derivative; and
d) removing any additional protecting group which could be present if desired, or if necessary.

2. The process of Claim 1 wherein B is independently present twice and Z is O.

3. The process of Claim 2 wherein J is $NH_2$, and Q is

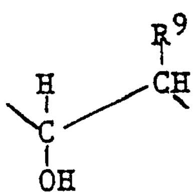

4. The process of Claim 2 wherein J is $NH_2$, and Q is

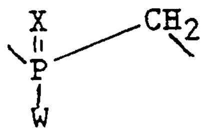

.

5. The process of Claim 2 wherein J is $NH_2$, and Q is

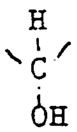

.

6. The process of Claim 1 wherein B is present once and Z is O.

7. The process of Claim 6 wherein Q is

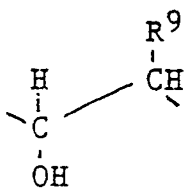

.

8. The process of Claim 6 wherein Q is

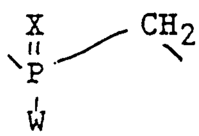

.

9. The process of Claim 6 wherein Q is

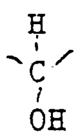

.

10. The process of Claim 1 wherein B is always absent.

11. The process of Claim 10 wherein Q is

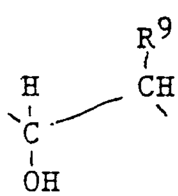

.

12. The process of Claim 10 wherein Q is

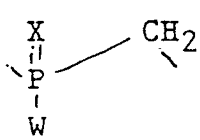

.

13. The process of Claim 10 wherein Q is

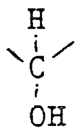

.

14. The process of Claim 1 wherein G is:

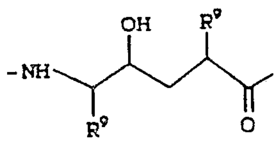 or 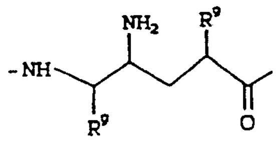 .

15. The process of Claim 1 wherein G is:

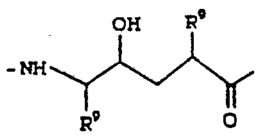 or 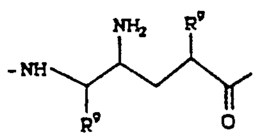 ; and

and B is absent or present once.

16. The process of Claim 1 wherein G is

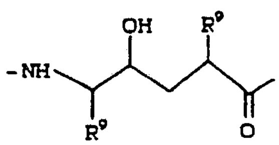 or 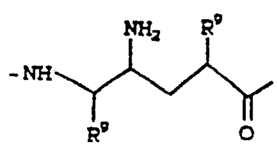 ;

B is absent or present once; and

J is 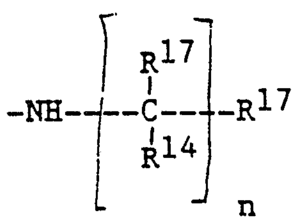 .

17. The process of Claim 1 wherein:

A is

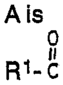 - or $R^1$-$SO_2$-, with the proviso that $R^1$ is not aryl when attached to S;

G is:

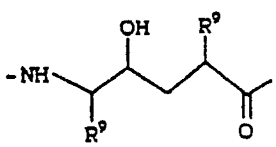 or 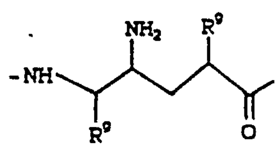 ;

B is absent or present once; and

J is 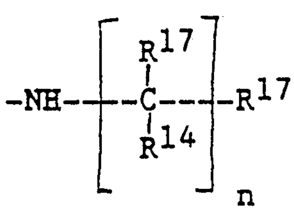 .

18.- The process of claim 1, wherein the compound obtained is N′-(1,1-dimethylethylaminocarbonyl)-5(S)-amino-4(S)-hydroxy-6-phenyl-2(R)-(phenylmethyl)hexanoyl-leucyl-phenylalanylamide, or pharmaceutically acceptable salt thereof.

19. The process of claim 1, wherein the compound obtained is N′-(2,2-dimethylpropanoyl)-5(S)-amino-4(S)-hydroxy-6-phenyl-2(R)-(phenylmethyl)hexanoyl-leucyl-phenylalanylamide, or pharmaceutically acceptable salt thereof.

20.- The process of claim 1, wherein the compound obtained is N′-(2-thienoyl)-5(S)amino-4(S)-hydroxy-6-phenyl-2(R)-(phenylmethyl)hexanoyl-leucyl-phenylalanylamide, or pharmaceutically acceptable salt thereof.

21. The process of claim 1, wherein the compound obtained is N′-(3,3-dimethylbutanoyl)-5(S)-amino-4(S)-hydroxy-6-phenyl-2(R)-(phenylmethyl)hexanoyl-leucyl-phenylalanylamide, or pharmaceutically acceptable salt thereof.

22. The process of claim 1, wherein the compound obtained is N′-(3-phenylpropanoyl)-5(S)-amino-4(S)-hydroxy-6-phenyl-2(R)-(phenylmethyl)hexanoyl-leucyl-phenylalanylamide, or pharmaceutically acceptable salt thereof.

23. The process of claim 1, wherein the compound obtained is selected from N-benzyl-N′-(succinoyl)-5(S)-amino-4(S)-hydroxy-6-phenyl-2(R)-(phenylmethyl)hexanoyl isoleucyl amide;
N-(2(R)-hydroxy-1(S)-indanyl)-N′-(succinoyl)-5(S)-amino-4(S)-hydroxy-6-phenyl-2(R)-(phenylmethyl)hexanamide;
N-(2(R)-hydroxy-1(S)-indanyl)-N′-(methanesulfonyl)-5(S)-amino-4(S)-hydroxy-6-phenyl-2(R)-(phenylmethyl)hexanamide;
N-(2(R)-hydroxy-1(S)-indanyl)-N′-(5-oxo-2(S)-tetrahydrofurancarbonyl)-5(S)-amino-4(S)-hydroxy-6-phenyl-2(R)-(phenylmethyl)hexanamide,
N′-(2,2-dimethylpropanoyl)-5(S)-amino-4(S)-hydroxy-6-phenyl-2(R)-(phenylmethyl) hexanoyl-Leu-Phe-amide,
N′-(1,1-dimethylethylaminocarbonyl)-5(S)-amino-4(S)-hydroxy-2(R)-phenylmethyl-hexanoyl -Leucyl-Phenylalanyl-amide,
N′-(3-phenylpropanoyl)-5(S)-amino4(S)-hydroxy-6-phenyl-2(R)-(Phenylmethyl)-hexanoyl-Leucyl-Phenylalanyl-amide,
N′-(Succinoyl)-5(S)-amino-4(S)-hydroxy-6-phenyl-2(R)-(phenylmethyl)hexanoyl-N-(phenylmethyl)-Ile-amide,
N-(2(R)-hydroxy-1(S)-indanyl)-N′-(4-t-butyldimethylsilyloxybutanoyl)-5(S)-amino-4(S)-hydroxy-6-phenyl-2(R)-(phenylmethyl)hexanamide,
N-(2(R)-hydroxy-1(S)-indanyl)-N′-(methanesulfonyl)-5(S)-amino-4(S)-hydroxy-6-phenyl-2(R)-(phenylmethyl)hexanamide,
N-(2(R)-hydroxy-1(S)-indanyl)-N′-(4-hydroxybutanoyl)-5(S)-amino-4(S)-hydroxy-6-phenyl-2(R)-phenylmethyl hexanamide,
N′-(methanesulfonyl)-5(S)-amino-4(S)-hydroxy-6-phenyl-2(R)-(phenylmethyl)hexanoyl-N-[2,3-dihydroxypropyl]-Val-amide, or
N-(2(R)-hydroxy-1(S)-indanyl)-N′-[2-(2-[2-methoxyethoxy]ethoxy)ethoxycarbonyl]-5(S)-amino-2(R)-benzyl-4(S)-hydroxy-6-phenyl hexanamide,
or pharmaceutically acceptable salts thereof.